Europäisches Patentamt

European Patent Office

Office européen des brevets

(19)

(11) Publication number: **0 092 388 B2**

(12) **NEW EUROPEAN PATENT SPECIFICATION**

(45) Date of publication of the new patent specification:
26.10.94

(51) Int. Cl.5: **C12N 15/76**, C12N 1/20,
//C12R1/19,C12R1/465

(21) Application number: **83302116.5**

(22) Date of filing: **14.04.83**

(54) **Cloning vectors.**

(30) Priority: **16.04.82 US 368947**

(43) Date of publication of application:
**26.10.83 Bulletin 83/43**

(45) Publication of the grant of the patent:
**04.07.90 Bulletin 90/27**

(45) Mention of the opposition decision:
**26.10.94 Bulletin 94/43**

(84) Designated Contracting States:
**AT BE CH DE FR GB IT LI NL SE**

(56) References cited:

CHEMICAL ABSTRACTS, vol. 94, no.15, April 13, 1981, page 358, abstract 117613j, Colombus, Ohio, US; M.J.BIBB et al.:"Development of cloning system for Streptomyces"

NATURE, vol. 274, July 27, 1978, pp. 398-400; Macmillan Journals Ltd., M.J. BIBB et al.: "Transformations of plasmid DNA into Streptomyces at high frequency"

(73) Proprietor: **ELI LILLY AND COMPANY**
**307, East McCarty Street**
**Indianapolis Indiana 46285 (US)**

(72) Inventor: **Hershberger, Charles Lee**
**Rural Route No. 1**
**Box 343**
**New Palestine Indiana 46163 (US)**
Inventor: **Larson, Jeffrey Lynn**
**7023 Mikesell Drive**
**Indianapolis Indiana 46260 (US)**

(74) Representative: **Hudson, Christopher Mark et al**
**Erl Wood Manor**
**Windlesham Surrey GU20 6PH (GB)**

EP 0 092 388 B2

NATURE, vol. 284, April 10, 1980, pp. 528-531, Macmillan Journals Ltd., M. BIBB et al.: "A DNA cloning system for interspecies gene transfer in antibiotic-producing Streptomyces"

JOURNAL OF BACTERIOLOGY, vol. 146, no.1, April 1981, pp. 360-368, J.L. SCHOTTEL et al.: "Cloning and expression in Streptomyces lividans of antibiotic resistance genes derived from Escherichia coli"

JOURNAL OF BACTERIOLOGY, vol. 131, no. 1, July 1977, p. 251-258, SCHREMPF, H. and GOEBEL, W.: "Characterization of a plasmid from Streptomyces coelicolor"

GENETIC ENGINEERING (eds. H.W. Boyer, S. Nicosia), Elsevier, North Holland, Biomedical Press (1978), p. 47-58, GOEBEL, W et al.: "Replication and gene expression of extrachromosomal replicons in unnatural bacterial hosts"

**Description**

The present invention relates to selectable novel recombinant DNA cloning vectors. The invention also relates to transformants and a method for detecting transformants of the aforementioned vectors.

The vectors of this invention can be transformed and selected in Streptomyces or E. coli. As Schottel, J. L. et al. (1981, J. of Bacteriology 146, No. 1, pages 360-368) point out plasmids able to replicate in both E. coli and Streptomyces are particularly useful because difficulties encountered in working with Streptomyces spp in general. Since over half of the clinically important antibiotics are produced by Streptomyces strains, it is desirable to develop cloning systems and vectors that are applicable to that industrially important group. Such vectors allow for the cloning of genes into Streptomyces both for increasing the yields of known antibiotics as well as for the production of new antibiotics and antibiotic derivatives.

Bibb, M. et al. (1980, Nature 284, pages 526-531 at page 529 teach that in plasmids SCP2 and SCP2* the origin of replication functional in Streptomyces lies within the ~ 15 kb BamHI-PstI restriction fragment of those plasmids and that the whole of that fragment is required to conserve Streptomyces functionality when using the fragment in cloning.

Schrempf, H. et al. (1977, J. of Bacteriology, 131, No. 1, pages 251-258) characterises plasmid pSH1 of Streptomyces coelicolor which is the equivalent of plasmid SCP2. The reference identifies some of the restriction enzyme sites on this plasmid. It is suggested that the EcoRI and HindIII restriction enzyme sites may be useful in cloning DNA, but the location of the origin of replication was not known.

Goebel, W. et al. (1978, Genetic Engineering, Elsevier, North Holland Biomedical Press, pages 47 to 58) describes the stability of hybrid plasmids consisting of an Escherichia coli vector and Streptomyces DNA. The reference discloses the cloning of an EcoRI-digested plasmid from E.coli into the EcoRI site of plasmid pSH1 (SCP2).

It has now been discovered that the origin of replication functional in Streptomyces is located in the ~5.4 kb EcoRI-SalI restriction fragment of plasmids SCP2 and SCP2* and that the use of the ~5.4 kb fragment or the ~6.0 kb SalI restriction fragment, in cloning is sufficient to conserve functionality in Streptomyces. The discovery permits construction of smaller, more versatile plasmids.

The method of the present invention also provides for the convenient selection of transformants. Since transformation is a very low frequency event, such a functional test is a practical necessity for determining which cell(s), of among the millions of cells, has acquired the plasmid DNA. This is important because DNA sequences that are non-selectable can be inserted into the vectors and, upon transformation, cells containing the vector and the particular DNA sequence of interest can be isolated by appropriate phenotypic selection.

For purposes of the present invention as disclosed and claimed herein, the following terms are as defined below.

Plasmid pLR1 or pLR4 3.4kb BamHI Restriction Fragment - the same 3.4kb BamHI neomycin resistance-conferring fragment contained in plasmid pIJ2.

| | |
|---|---|
| Amp$^R$ | - the ampicillin resistant phenotype. |
| Amp$^S$ | - the ampicillin sensitive phenotype. |
| Tet$^R$ | - the tetracycline resistant phenotype. |
| Tet$^S$ | - the tetracycline sensitive phenotype. |
| CM$^R$ | - the chloramphenicol resistant phenotype. |
| CM$^S$ | - the chloramphenicol sensitive phenotype. |
| Neo$^R$ | - the neomycin resistant phenotype. |
| Neo$^S$ | - the neomycin sensitive phenotype. |
| Thio$^R$ | __ the thiostrepton resistant phenotype. |
| Thio$^S$ | - the thiostrepton sensitive phenotype. |

Neo$^R$, Neo$^S$, Thio$^R$, and Thio$^S$ refer only to results of tests in Streptomyces as used in this disclosure. Amp$^R$, Amp$^S$, Tet$^R$, Tet$^S$, Cm$^R$ and Cm$^S$ refer only to results of tests in E. coli as used in this disclosure.

The present invention specifically relates to a recombinant DNA cloning vector which has as the only restriction fragment derived from plasmid SCP2 or SCP2* a ~5.4 kb EcoRI-SalI or ~6.0 kb SalI restriction fragment comprising an origin of replication functional in Streptomyces, and which further comprises

a) a restriction fragment comprising the functional origin of a multi-copy E. coli plasmid,

b) one or more DNA segments that confer resistance to at least one antibiotic when transformed into a cell of E. coli, said cell being sensitive to the antibiotic for which resistance is conferred, and

c) one or more DNA segments that confer resistance to at least one antibiotic when transformed into a cell of Streptomyces, said cell being sensitive to the antibiotic for which resistance is conferred.

The recombinant DNA cloning vectors are made by a process which comprises ligating, the ~5.4 kb EcoRI-SalI or ~6.0 kb SalI restriction fragment and

a) a restriction fragment comprising the functional origin of a multi-copy E. coli plasmid,

b) one or more DNA segments that confer resistance to at least one antibiotic when transformed into a cell of E. coli, said cell being sensitive to the antibiotic for which resistance is conferred, and

c) one or more DNA segments that confer resistance to at least one antibiotic when transformed into a cell of Streptomyces, said cell being sensitive to the antibiotic for which resistance is conferred.

The invention comprises transformants comprising the above recombinant DNA cloning vector.

The invention further comprises a method for detecting transformants comprising:

1) mixing Streptomyces cells, under transforming conditions, with a recombinant DNA cloning vector, said vector comprising

a) an origin of replication and P gene-containing restriction fragment of plasmid SCP2 or SCP2*, and

b) a non-lethal DNA sequence cloned into the EcoRI restriction site of said P gene, and

2) growing said Streptomyces cells on a lawn of an indicator Streptomyces strain and selecting colonies that show the M pock phenotype.

Vectors can be constructed by ligating an origin of replication- containing and Streptomyces tra function-conferring restriction fragment of plasmid SCP2 or SCP2* into an E. coli origin of replication-containing and antibiotic resistance-conferring restriction fragment of an E. coli plasmid. Plasmids SCP2 and SCP2*, from which origins of replication are constructed, are each ~31kb and show similar restriction patterns. Plasmid SCP2* arose as a spontaneous mutant of plasmid SCP2 and codes for a selectable colony pock morphology. Although the pock is distinguishable from that of plasmid SCP2, in other ways plasmids SCP2 and SCP2* are virtually identical.

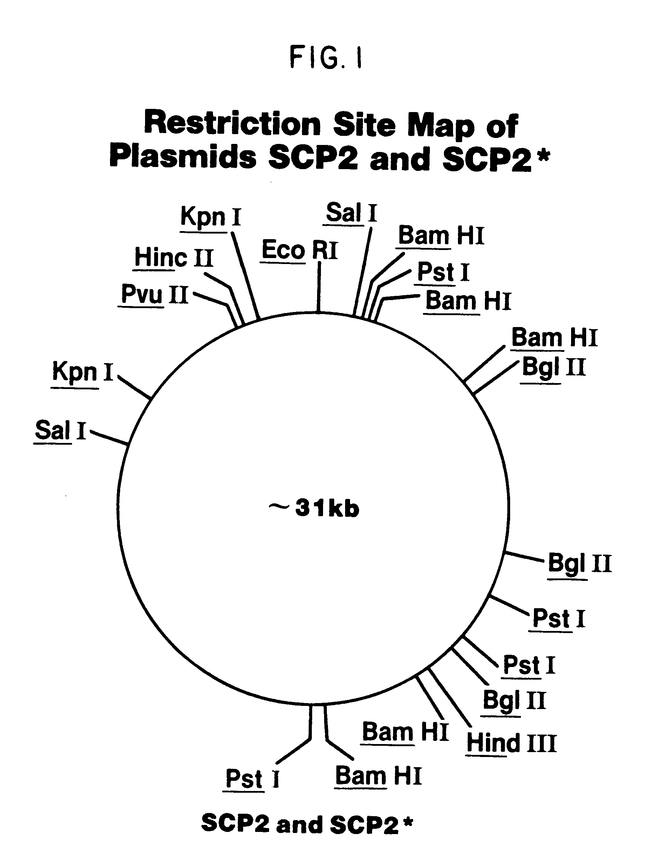

Since the present disclosure teaches that the Streptomyces tra function and the origin of replication of plasmids SCP2 and SCP2* are within their respective ~5.4 kb EcoRI-SalI restriction fragments, a variety of different origin of replication-containing and Streptomyces tra function-conferring fragments can be generated. This is accomplished by digestion with restriction enzymes that cut outside the ~5.4 kb EcoRI SalI region. A detailed restriction site map of plasmid SCP2* (and thus also plasmid SCP2) is presented in Figure 1 of the accompanying drawings.

Plasmids SCP2 and SCP2* can be conventionally isolated respectively from the Streptomyces coelicolor A3(2) and Streptomyces coelicolor M110 strains deposited and made part of the permanent stock culture collection of the Northern Regional Research Laboratory, Peoria, Illinois. Streptomyces coelicolor A3(2) is available to the public as a preferred source and stock reservoir of plasmid SCP2 under the accession number 15042. Streptomyces coelicolor M110 is available to the public as a preferred source and stock reservoir of plasmid SCP2* under the accession number 15041.

Many tra function-conferring and origin of replication-containing restriction fragments of plasmids SCP2 and SCP2* can be constructed. Those specifically exemplified, for illustrative purposes, include the ~5.4 kb EcoRI-SalI, the ~6.0 kb SalI, the ~19 kb EcoRI-HindIII, and the ~31 kb EcoRI restriction fragments of plasmid SCP2* and the ~31 BglII restriction fragment of plasmid SCP2. The aforementioned plasmid SCP2* and SCP2 fragments were respectively ligated to an origin of replication-containing and antibiotic resistance-conferring fragment of E. coli plasmids pBR325 and pBR322. Those skilled in the art will recognize that although not required, it is convenient for both the DNA segment that confers antibiotic resistance in E. coli and the E. coli origin of replication to comprise a restriction fragment of the same E. coli plasmid.

Thus, for convenience and ease of construction, the ~31 kb EcoRI fragment of plasmid SCP2* and the ~6 kb EcoRI fragment of plasmid pBR325 were ligated to form illustrative plasmids pJL120 and pJL121. Recombinant plasmids of two orientations result because the fragments can be ligated in either direction. Similarly, ligation of the SCP2* ~6.0 kb SalI fragment and the ~6 kb SalI fragment of pBR325 results in the illustrative plasmids pJL180 and pJL181; ligation of the SCP2* ~5.4 kb EcoRI.SalI fragment and the ~4.8 kb EcoRI-SalI fragment of pBR325 results in the illustrative plasmid pJL125.

All of the aforementioned vectors are readily selectable in each of E. coli and Streptomyces. For example, in E. coli, plasmids pJL120 and pJL121 confer ampicillin and tetracycline resistance; plasmids pJL180 and pJL181 confer ampicillin and chloramphenicol resistance; and plasmid pJL125 confers only ampicillin resistance. Therefore, the vectors are conventionally selectable in the E. coli host system by adding the appropriate antibiotic to the culture medium.

The aforementioned vectors also produce the 'pock' phenotype and therefore are conventionally selectable in Streptomyces. The 'pock' phenotype is an assayable trait and known phenomenon (Bibb and Hopwood, 1981, J. Gen. Microbiol. 126:427) associated with lethal zygosis and the tra function (tra = genes coding for sexual transmissability) of Streptomyces sex factors. Three distinct 'pock' morphologies are associated with transformants, when plated on an appropriate indicator strain, of plasmids SCP2, SCP2*, and SCP2 and SCP2* derivatives. The colony morphology identified with the wild-type SCP2 and the mutant SCP2* are respectively designated herein as P and P*. A third and heretofore unknown pock morphology results from cloning into the *Eco*RI restriction site of SCP2 or SCP2*. Such an insertion inactivates the P gene and unexpectedly results in a morphologically distinguishable minipock phenotype, designated herein as M, when transformants are appropriately plated. "Minipock" is a pock of significantly smaller size than pocks caused by either SCP2 or SCP2*.

Only transformed *Streptomyces* cells will show the M pock phenotype and therefore transformants can be readily identified and selected. Those skilled in the art will quickly recognize, from the above description of the present pJL vectors, that plasmids pJL120, pJL121, and pJL125 code for M and phenotype, that plasmids pJL180 and pJL181 code for P* phenotype. Appropriate indicator strains for expression of the pock phenotype are known and include the various SCP2⁻ and SCP2*⁻ strains as illustrated in the Examples below. The present vectors are thus selectable and extremely useful in *Streptomyces.*

The aforementioned plasmids can also be provided with a DNA segment that confers antibiotic resistance in *Streptomyces.* Such derivatives, specifically exemplified for illustrative purposes by plasmids pJL190 and pJL195, express an additional selectable phenotype. Plasmid pJL190 was constructed by ligating the neomycin resistance-conferring ~7.7 kb *Eco*RI-*Hind*III fragment of plasmid pLR4 to the ~19 kb *Eco*RI-*Hind*III fragment of plasmid pJL121. Plasmid pJL195 was constructed by ligating the pLR4 ~7.5 kb *Edo*RI-partial *Sal*I fragment to the ~5.4 kb *Eco*RI-*Sal*I fragment of plasmid pJL125. The latter pJL125 plasmid comprises the largest (5.4 kb) *EcoRISal*I fragment of plasmid pSCP2* and was constructed by *Sal*I deletion of plasmid pJL121. Illustrative plasmids pJL190 and pJL195, in addition to neomycin resistance, also express the M phenotype as discussed above.

Plasmid pLR4, the source of the neomycin resistance conferring fragments, is ~7.7 kb and is constructed by ligating *Bam*HI-treated plasmids pBR322 and pLR1. Plasmid pLR1 is ~14.8 kb and is constructed by ligating *Hind*III-treated plasmid pIJ2, disclosed in Thompson *et al.,* 1980, Nature 286:525, to *HindIII*-treated plasmid pBR322. As is readily apparent to those skilled in the art, both plasmids pLR4 and pLR1 contain the same neomycin resistance gene and thus either plasmid can be used for constructing the aforementioned pJL neomycin resistance vectors.

An additional neomycin resistance-conferring plasmid, designated as pJL192, was isolated as a spontaneous mutant of plasmid pJL190 resident in *Streptomyces griseofuscus.* Plasmid pJL192 specifies resistance to elevated levels of neomycin and therefore comprises a novel neomycin resistance gene which is distinguishable from the resistance gene comprising plasmids pJL190, pJL195, pIJ2, pLR4, and pLR1. In a similar manner, an additional neomycin resistance-conferring plasmid, designated as pJL199, as isolated as a spontaneous mutant of plasmid pJL195. Those skilled in the art will recognize that the novel neomycin resistance gene of plasmid pJL192 or pJL199 can be readily excised and ligated to other vectors. The gene allows for improved and more efficient selection of transformants. As in the case of plasmids pJL190 and pJL195, transformants of plasmids pJL192 and pJL199 express the M phenotype when plated on an appropriate indicator strain.

Plasmid pJL192 can be conventionally isolated from *E. coli* K12 C600$R_k$—$M_k$-/pJL192, a strain deposited and made part of the permanent stock culture collection of the Northern Regional Research Laboratory, Peoria, Illinois. It is available to the public as a stock reservoir and preferred source of plasmid pJL192 under the accession number B-15040.

A DNA segment that confers resistance to antibiotic thiostrepton, exemplified by the ~1.35 kb *Bam*HI restriction fragment of plasmid pLR2, can also be used with or substituted for the neomycin resistance-conferring segment. Plasmid pLR2, the souce of the thiostrepton resistance conferring fragment, is ~18.7 kb and is constructed by ligating *Hind*III treated plasmid pIJ6, disclosed in Thompson *et al.,* 1980, Nature 286:525, to *Hind*III treated plasmid pBR322. Plasmid pLR2 is functional in *E. coli* and therefore can be amplified and isolated conveniently for subsequent manipulation.

For convenience and ease of construction, the thiostrepton resistance conferring ~1.35 kb *Bam*HI fragment of plasmid pLR2 was ligated into the *Bam*HI restriction site of plasmid pBR328 to form plasmid pJL193. The ~1 kb *Bcl*I restriction fragment of PJL193 contains the thiostrepton resistance-conferring DNA segment. Therefore, ligation as described in Examples 52-56, results in vectors that are within the scope of the present invention.

Various plasmids SCP2 and SCP2* restriction fragments can be used for purposes of constructing the present invention provided that the origin of replication contained in their respective ~5.4kb EcoRI-SalI restriction fragments is present. Such additional plasmid SCP2 and SCP2* restriction fragments include, but are not limited to, the ~6kb SalI, ~15kb PstI, ~23kb BglII, ~15kb BamHI, ~14kb EcoRI-PstI, ~13kb EcoRI-BamHI, and ~15kb PstI-BamHI fragments. These fragments contain the Streptomyces tra function and can be ligated to a functional E. coli origin of replication-containing and antibiotic resistance-conferring restriction fragment of an E. coli plasmid. Such E. coli plasmids include, for example, plasmids pBR322, pBR324, pBR325, pBR327, pBR328 and the like. Therefore, the present invention is not limited to the use of either plasmid pBR322 or pBR325 as exemplified in several pJL constructions.

Although the neomycin and thiostrepton antibiotic resistance-conferring DNA segments exemplified herein are respectively the ~7.7kb EcoRI-HindIII and the ~7.5kb EcoRI-partial SalI fragments of plasmid pLR4 and the pLR2 ~1.35 BamHI and the pJL193 ~1kb BclI fragments, those skilled in the art can construct and use other DNA segments that also confer resistance to neomycin or thiostrepton. Other neomycin resistance-conferring DNA segments of plasmid pLR1 include, for example, the ~3.4kb BamHI restriction fragment, the ~3.5kb PstI restriction fragment, and the larger of the SstI-KpnI subfragments of the ~3.4kb BamHI restriction fragment. Other thiostrepton resistance-conferring segments include, for example, the ~13kb PstI fragment of plasmid pLR2. still other DNA segments conferring resistance to the same or to different antibiotics such as, for example, hygromycin, viamycin, tylosin, erythromycin and the like, can also be constructed and used by those skilled in the art. In addition, various functional derivatives of the above described antibiotic resistance-conferring DNA segments can be constructed by adding, eliminating, or substituting nucleotides in accordance with the genetic code.

Ligation of the aforementioned derivatives, or any of the other antibiotic resistance-conferring DNA segments, to a vector comprising an E coli antibiotic resistance-conferring DNA segment, an E. coli origin of replication-containing restriction fragment, and also an origin of replication-containing restriction fragment of plasmids SCP2 or SCP2*, is possible.

Therefore, an antibiotic resistance-conferring DNA segment can be used as a selectable marker in place of the Streptomyces tra function and associated pock phenotype. Thus, the vectors are not limited to the use of tra alone or in combination with an antibiotic resistance-conferring DNA segment. In addition, a particular antibiotic resistance-conferring DNA segment is not limited to a single position on the present chimeric plasmids but can be ligated or inserted at varying sites provided that an origin of replication or other critical plasmid controlled physiological functions are not disrupted. Those skilled in the art understand or can readily determine which sites are advantageous for ligation or insertion of a particular DNA segment.

The various restriction fragments of plasmids SCP2, SCP2*, pBR325, pBR322 and the like, and also the various antibiotic resistance-conferring DNA segments comprising the present vectors, can be modified to facilitate ligation. For example, molecular linkers can be provided to some or all of the aforementioned DNA fragments. Thus, specific sites for subsequent ligation can be constructed conveniently. In addition, the origin of replication-containing restriction fragments can also be modified by adding, eliminating, or substituting certain nucleotides to alter characteristics and to provide a variety of restriction sites for ligation of DNA. Those skilled in the art understand nucleotide chemistry and the genetic code and thus which nucleotides are interchangeable and which DNA modifications are desirable for a specific purpose.

The recombinant DNA cloning vectors that contain the SCP2 or SCP2* *Streptomyces tra* function are self transmissable and thus readily transferred during mating between transformed and non-transformed *Streptomyces* taxa. This is advanteous because the present vectors therefore can be transformed not only by protoplast transformation but also by conventional genetic crosses. Consequently, the vectors are useful in *Streptomyces* strains which are difficult to protoplast thus greatly expanding the number of hosts in which genetic manipulation and DNA cloning can be done.

More importantly, DNA-libraries constructed in the present vectors can be conveniently and rapidly screened, for interesting genes by conventional replica-plate mating procedures. Without the *tra* function, DNA must be isolated from each of the thousands of clones in the library and transformed into appropriate strains to identify clones that contain desirable genes. Since there are no broadly applicable phage vectors for use in *Streptomyces* the present *tra*+ vectors fulfill the general cloning and screening role analogous to that of bacteriophge λ in replica-plate transduction for screening gene libraries in *E. coli.* Desirable genes can thus be readily identified by the replica-plate mating procedure and then easily amplified by shuttling into *E. coli* as described in Example 20C below.

The vectors of the present invention are broadly applicable and are transformed into host cells of many *Streptomyces* taxa, particularly restrictionless strains of economically important taxa that produce antibiotics such as aminoglycoside, macrolide, β-lactam, polyether, and glycopeptide antibiotics. Such restrictionless strains are readily selected and isolated from *Streptomyces* taxa by conventional procedures well known in the art (Lomovskaya *et al.,* 1980, Microbiological Reviews 44:206). Host cells of restrictionless strains lack restriction enzymes and therfore do not cut or degrade plasmid DNA upon transformation. For purposes of the present application, host cells containing restriction enzymes that do not cut any of the restriction sites of the present vectors are also considered restrictionless.

Preferred host cells of restrictionless strains of *Streptomyces* taxa that produce aminoglycoside antibiotics and in which the present vectors are especially useful and are transformed, include restrictionless cells of, for example: *S. kana—myceticus* (Kanamycins), *S. chrestomyceticus* (aminosidine), *S. griseoflavus* (antibiotic MA 1267), *S. microsporeus* (Antibiotic SF-767), *S, ribosidificus* (antibiotic SF733), *S. flavopersicus* (spectinomycin), *S. spectabilis* (actinospectacin), *S. rimosus* forma *paromomycinus* - (paromomycins, catenulin), *S. fradiae* var. *italicus* (aminosidine), *S. blueinsis* var. *bluensis* (bluensomycin), *S. catenulae* (catenulin), *S. olivoreticuli* var. *cellulophilus* (destomycin A), *S. tenebrarius* (tobramycin, apramycin), *S. lavendulae* (neomycin), *S. albogriseolus* (neomycins, *S. alblus* var. *metamycinus* - (metamycin), *S. hygroscopicus* var. *sagamiensis* (spectinomycin), *S. bikiniensis* (streptomycin), *S. griseus* (streptomycin), *S. erythrochromogenes* var. *narutoensis* (streptomycin), *S. poolensis* (streptomycin), *S. galbus* (streptomycin), *S. rameus* (streptomycin), *S. olivaceus* (streptomycin), *S. mascheunsis* - (streptomycin), *S. hygroscopicus* var. *limoneus* (validamycins), *S. rimofaciens* (destomycins), *S. hygroscopicus* forma *glebosus* (glebomycin), *S. fradiae* (hybrimycins neomycins), *S. eurocidicus* (antibiotic A16316-C), *S. aquacanus* (N-methyl hygromycin B), *S. crystallinus* (hygromycin A), *S. noboritoensis* - (hygromycin), *S. hygroscopicus* (hygromycins), *S. atrofaciens* (hygromycin), *S. kasugaspinus* - (kasugamycins), *S. kasugaensis* (kasugamycins), *S. netropsis* (antibiotic LL-AM31), *S, lividus* - (lividomycins), *S. hofuensis* (seldomycin complex), and *S. canus* (ribosyl paromamine).

Preferred host cells of restrictionless strains of *Streptomyces* taxa that produce macrolide antibiotics and in which the present vectors are especially useful and are transformed, include restrictionless cells of, for example: *S. caelestis* (antibiotic M188), *S. platensis* (platenomycin), *S. rochei* var. *volubilis* (antibiotic T2636), *S. venezuelae* (methymycins), *S. griseofuscus* (bundlin), *S. narbonensis* (josamycin, narbomycin), *S. funicidicus* (antibiotic NA-181), *S. griseofaciens* (antibiotic PA133A, B), *S. roseocitreus* (albocycline), *S. bruneogriseus* (albocycline), *S. roseochromogenes* (albocycline), *S. cinerochromogenes* (cineromycin B), *S. albus* (albomycetin), *S. felleus* (argomycin, picromycin), *S. rochei* (lankacidin, borrellidin), *S. violaceoniger* (lankacidin), *S. griseus* (borrelidin), *S. maizeus* (ingramycin), *S. albus* var. *coilmyceticus* - (coleimycin), *S. mycarofaciens* (acetyl-leukomycin, espinomycin), *S. hygroscopicus* (turimycin, relomycin, maridomycin, tylosin, carbomycin), *S. griseospiralis* (relomycin), *S. lavendulae* (aldgamycin), *S. rimosus* - (neutramycin), *S. deltae* (deltamycins), *S. fungicidicus* var. *espinomyceticus* (espinomycins), *S. fur—dicidicus (mydeca*-mycin), *S. eurocidicus* (methymycin), *S. griseolus* (griseomycin), *S. flavoch—romogenes* (amaromycin, shincomycins), *S. fimbriatus* (amaromycin), *S. fasciculus* (amaromycin), *S. erythreus* (erythromycins), *S. antibioticus* (oleandomycin), *S. olivochromogenes* (oleandomycin), *S. spinichromogenes* var. *suragaoensis* (kujimycins), *S. kitasatoensis* (leucomycin), *S. narbonensis* var. *josamyceticus* (leucomycin A3, josamycin), *S. albogriseolus* (mikonomycin), *S. bikiniensis* (chalcomycin), *s. cirratus* (cirramycin), *S. djakartensis* (niddamycin), *S. eurythermus* (angolamycin), *S. fradiae* (tylosin, lacetnocin, macrocin), *S. goshikiensis* (bandamycin), *S. griseoflavus* (acumycin), *S. halstedii* - (carbomycin), *S. tendae* (carbomycin), *S. macrosporeus* (carbomycin), *S. thermotolerans* (carbomycin), *S. albireticuli* (carbomycin), and *S. ambofaciens* (spiramycin).

Preferred host cells of restrictionless strains of *Streptomyces* taxa that produce $\beta$-lactam antibiotics and in which the present vectors are especially useful and are transformed, include restrictionless cells of, for example: *S. lipmanii* (A16884, MM4550, MM13902), *S. clavuligerus* (A16886B, clavulanic acid), *S. lactamdurans* (cephamycin C), *S. griseus* (cephamycin A, B), *S. hygroscopicins* (deacetoxycephalosporin C), *S. wadayamensis* (WS-3442-D), *S. chartreusis* (SF 1623), *S. heteromorphus* and *S. panayensis* - (C2081X); *S. cinnamonensis, S. fimbriatus, S. halsledii, S. rochei* and *S. viridochromogenes* - (cephamycins A, B); *S. cattleya* (thienamycin); and *S. olivaceus, S. flavovirens, S. flavus, S. fulvoviridis, S. argenteolus,* and *S. sioyaensis* (MM4550 and MM13902).

Preferred host cells of restrictionless strains of *Streptomyces* taxa that produce polyether antibiotics and in which the present vectors are especially useful and are transformed, include restrictionless cells of, for example: *S. albus* (A204, A28695A and B, salinomycin), *S. hygroscopicus* (A218, emericid, DE3936), A120A, A2895A and B, etheromycin, dianemycin), *S. griseus* (grisorixin), *S. conglobatus* (ionomycin), *S. eurocidicus* var. *asterocidicus* (laidlomycin), *S. lasaliensis* (lasalocid) *S. ribosidificus* (lonomycin), *S. cacaoi* var. *asoensis* (lysocellin), *S. cinnamonensis* (monensin), *S. aureofaciens* (narasin), *S. gallinarius* - (RP 30504), *S. longwoodensis* (lysocellin), *S. flaveolus* (CP38936), *S. mutabilis* (S-11743a), and *S. violaceoniger* (nigericin).

Preferred host cells of restrictionless strains of *Streptomyces* taxa that produce glycopeptide antibiotics and in which the present vectors are especially useful and are transformed, include restrictionless cells of, for example: *S. orientalis* and *S. haranomachiensis* (vancomycin); *S. candidus* (A-35512, avoparcin), and *S. eburosporeus* (LL-AM 374).

Preferred host cells of other *Streptomyces* restrictionless strains in which the present vectors are especially useful and can be transformed, include restrictionless cells of, for example: *S. granuloruber, S. roseosporus, S. lividans, S. espinosus,* and *S. azureus.*

In addition to the representative *Streptomyces* host cells described above, the present vectors are also useful and can be transformed into *E. coli.* Thus, vectors of the present invention have wide application and are useful and can be transformed into host cells of a variety of organisms.

While all the embodiments of the present invention are useful, some of the present recombinant DNA cloning vectors and transformants are preferred. Accordingly, preferred vectors are pJL114, pJL121, pJL125, pJL180, pJL190, pJL192, pJL195, pJL197, pJL199 and pHJL212 and preferred transformants are *Streptomyces griseofuscus*/pJL114, S. *griseofuscus*/pJL121, S. *griseofuscus*/pJL125, S. *griseofuscus*/pJL180, S. *griseofuscus*/pJL190, S. *griseofuscus*/pJL192, S. *griseofuscus*/pJL195, S. *griseofuscus*/pJL199, S. *griseofuscus*/pJL197, S. *griseofuscus*/pHJL212, *E. coli* K12 C600$R_k$—$M_k$-/pJL114, *E. coli* K12C600$R_k$—$M_k$-/pJL121, *E. coli* K12 C600$R_k$—$M_k$-/pJL125, *E. coli* K12 C600$R_k$—$M_k$-/pJL180, *E. coli* K12 C600$R_k$—$M_k$-/pJL190, *E. coli* K12 C600$R_k$—$M_k$-/pJL192, *E. coli* K12 C600$R_k$—$M_k$-/pJL195, *E. coli* K12 C600$R_k$—$M_k$-/pJL199, *E. coli* K12 C600$R_k$—$M_k$-/pJL197, and *E. coli* K12 C600$R_k$—$M_k$-/pHJL212. Moreover, of this preferred group, plasmids pJL190, pJL192, pJL195, pJL197, pJL199 and pJL and transformants S. *griseofuscus*/pJL190, S. *griseofuscus*/pJL192, S. *griseofuscus*/pJL195, S. *griseofuscus*/pJL197, S. *griseofuscus*/pJL199, S. *griseofuscus*/pHJL212, *E. coli* K12 C600$R_k$—$M_k$-/pJL190, *E. coli* K12 C600$R_k$—$M_k$-/pJL192, *E. coli* K12 C600$R_k$—$M_k$-/pJL195 and *E. coli* K12 C600$R_k$—$M_k$-/pJL197, *E. coli* K12 C600$R_k$—$M_k$-/pJL199 and *E. coli* K12 C600$R_k$—$M_k$-/pHJL212 are most preferred. *Streptomyces griseofuscus* is a preferred host because it does not contain an endogenous plasmid or synthesize an antibiotic. Therefore, transformants of *S. griseofuscus* can be screened for clones that express genes for antibiotic synthesis.

The vectors of the present invention comprises origins of replication that are functional in *E. coli* and *Streptomyces* and therefore provide flexibility in the choice of hosts. Consequently, cloned DNA sequences can be shuttled into *E. coli* for construction of new plasmids, physical analysis, and for mapping of restriction sites and then shuttled back into *Streptomyces* for functional analysis and improvement of strains. This is particularly advantageous because amplification and manipulation of plasmids can be done faster and more conveniently in *E. coli* than in *Streptomyces.* For example, the present vectors can be amplified conventionally in *E. coli* K12 by growth with spectinomycin or chloramphenicol. This is not possible in the *Streptomyces* host system. In addition, since all the plasmid vectors contain resistance markers that are expressed in *E. coli* K12, recombinants are easily selected. Therefore, large amounts of plasmid DNA can be isolated conveniently and in a shorter time than that required for doing similar procedures in *Streptomyces.* Thus, after desired recombinant DNA procedures are accomplished in the *E. coli* host system, the particular *Streptomyces* DNA can be removed, reconstructed to plasmid form (if necessary), and then transformed into a *Streptomyces* host cell. Since the present vectors are fully selectable in *Streptomyces,* identification of recombinant clones can be done efficiently.

The recombinant DNA cloning vectors and transformants of the present invention have broad utility and help fill the need for suitable cloning vehicles for use in *Streptomyces* and *E. coli.* Moreover, the ability of the present vectors to confer a pock phenotype or resistance to antibiotics also provides a functional means for selecting transformants. This is important because of the practical necessity for determining and selecting the particular cells that have acquired vector DNA. Additional DNA segments, that lack functional tests for their presence, can also be inserted into the present vectors and then transformants containing the non-selectable DNA can be isolated by appropriate antibiotic or other phenotype selection. Such non-selectable DNA segments can be inserted at any site, except within regions necessary for plasmid function and replication, and include genes that specify antibiotic modification enzymes and regulatory genes of all types.

More particularly, a non-selectable DNA segment that comprises a gene can be inserted into a plasmid such as, for example, illustrative plasmid pJL192, at the internal *Bam*HI restriction site of the ~7.7 kb *Eco*RI-*Hind*III resistance-conferring fragment. Such an insertion inactivates the neomycin resistance gene and thus allows for the easy identification of *Streptomyces* transformants containing the recombinant plasmid. This is done by first selecting for M pock morphology and, secondarily, identifying those M transformants that are not resistant to neomycin. In a similar manner, insertion of a DNA segment into illustrative plasmid pJL180 at, for example, the unique *Pst*I restriction site, inactivates the ampicillin resistance gene. Thus, *E. coli* transformants carrying this recombinant plasmid an also be identified easily by first selecting for chloramphenicol resistance and, secondarily, identifying those chloramphenicol resistant transformants that are not resistant to ampicillin. Therefore, the ability to select for antibiotic resistance or other phenotypic markers in *Streptomyces* and *E. coli* allows for the efficient isolation of the extremely rare cells that contain the particular non-selectable DNA of interest.

The functional test for antibiotic resistance, as described above, can also be used to identify DNA segments that act as control elements and direct expression of an individual antibiotic resistance gene. Such segments, including but not limited to, promoters, attenuators, repressors, inducers, ribosomal binding sites, and the like, can be used to control the expression of other genes in cells of *Streptomyces* and *E. coli.*

The antibiotic resistance-conferring vectors of the present invention are also useful for insuring that linked DNA segments are stably maintained in host cells over many generations. These genes or DNA fragments, covalently linked to an antibiotic resistance-conferring fragment and propagated either in *Streptomyces* or *E. coli*, are maintained by exposing the transformants to levels of antibiotic that are toxic to non-transformed cells. Therefore, transformants that lose the vector, and consequently any covalently linked DNA, cannot grow and are eliminated from the culture. Thus, the vectors of the present invention can be used to maintain any DNA sequence of interest.

The cloning vectors and transformants of the present invention provide for the cloning of genes to improve yields of various products that are currently produced in *Streptomyces* and related cells. Examples of such products include, but are not limited to, Streptomycin, Tylosin, Cephalosporins, Actaplanin, Narasin, Monensin, Apramycin, Tobramycin, Erythromycin, and the like. The present invention also provides selectable vectors that are useful for cloning, characterizing, and reconstructing DNA sequences that code for commercially important proteins such s, for example, human insulin, human proinsulin, human growth hormone, bovine growth hormone, glucagon, interferon, and the like; for enzymatic functions in metabolic pathways leading to commercially important processes and compounds; or for control elements that improve gene expression. These desired DNA sequences include, but are not limited to, DNA that codes for enzymes that catalyze synthesis of derivatized antibiotics such as, for example, Streptomycin, Cephalosporin, Tylosin, Actaplanin, Narasin, Monensin, Apramycin, Tobramycin, and Erythromycin derivatives, or for enzymes that mediate and increase bioproduction of antibiotics or other products.

The capability of inserting, stabilizing, and shuttling the aforementioned DNA segments into *Streptomyces* and *E. coli* allows for easy recombinant genetic manipulation for increasing the yield and availability of antibiotics that are produced by *Streptomyces.* In addition, since the plasmid SCP2 or SCP2* origin of replication codes for low copy number, almost any DNA sequence, including those that are lethal when expressed from a high copy number plasmid, can be readily cloned into the present vectors and shuttled between *Streptomyces* and *E. coli.*

*Streptomyces* coelicolor A3(2) and *S. coelicolor* M110, as respective sources of plasmids SCP2 and SCP2*, can be cultured in a number of ways using any several different media. Carbohydrate sources which are preferred in a culture medium include, for example, molasses, glucose, dextrin, and glycerol, and nitrogen sources include, for example, soy flour, amino acid mixtures, and peptones. Nutrient inorganic salts are also incorporated and include the customary salts capable of yielding sodium, potassium, ammonia, calcium, phosphate, chloride, sulfate, and like ions. As is necessary for the growth and development of other microorganisms, essential trace elements are also added. Such trace elements are commonly supplied as impurities incidental to the addition of other constituents of the medium.

*Streptomyces coelicolor* M110 and *S. coelicolor* A3(2) are grown under aerobic culture conditions over a relatively wide pH range of about 5 to 9 at temperatures ranging from about 15° to 40°C. For production of plasmids SCP2 and SCP2* at highest copy number, however, it is desirable to start with a culture medium at a pH of about 7.2 and maintain a culture temperature of about 30°C. Culturing *Streptomyces coelicolor* M110 and *S. coelicolor* A3(2) under the aforementioned conditions, results in a reservoir of cells from which plasmids SCP2 and SP2* are respectively isolated conveniently by techniques well known in the art.

The following examples further illustrate and detail the invention disclosed herein. Both an explanation of and the actual procedures for constructing the invention are described where appropriate.

Example 1

Isolation of plasmid SCP2*

A. Culture of *Streptomyces coelicolor* M110

A vegetative inoculum of *Streptomyces coelicolor* M110 (NRRL 15041) was conventionally prepared by growing the strain under submerged aerobic conditions in 50 ml of sterilized trypticase soy broth†at 35 g/l in deionized water.

The trypticase soy broth inoculum was incubated for 48 hours at a temperature of 30°C. The 50 ml culture was then homogenized, transferred to 450 ml of sterilized YEMESG†† medium, and then incubated for at least 40, but not more than 65 hours, at 30°C. The pH was not adjusted. After incubation, the *Streptomyces coelicolor* M110 cells were ready for harvest and subsequent isolation of plasmid DNA.

B. Plasmid isolation

About 10 g (wet wgt) of *Streptomyces coelicolor* M110 cells were harvested by centrifugation (10 minutes, 4°C, 10,000 rpm) and then about 10 ml/g wet wgt cells of TES buffer (.01M Tris(hydroxymethyl)-aminoethane [tris], .001 M EDTA, 25% sucrose, pH 8) was added. The cells were vortexed into suspension followed by addition of 10 ml/g wet wgt cells of .25M EDTA, pH 8 and then 5 ml/g wet wgt cells of lysozyme (10 mg/ml in TES). After the mixture was incubated at 37°C for about 15 minutes, about 1.5 ml/g wet wgt cells of 20% SDS (sodium lauryl sulfate (BDH Chemicals Ltd. Poole, England), was added. The resultant mixture was allowed to stand at room temperature for 30 minutes, and then 5M NaCl was added to give a final concentration of 1M NaCl. After standing again at room temperature (15 minutes), the mixture was placed on ice for 2 hours. The lysate was centrifuged (20 minutes, 4°C, 17,500 rpm) and the supernatant was pooled and mixed with .64 volumes of isopropyl alcohol. The DNA precipitate was collected by centrifugation (15 minutes, 4°C, 10,000 rpm). The precipitate was air dried and then resuspended in 1 ml/g wet wgt cells of TE buffer (.01M Tris, .001M EDTA). Centrifugation (20 hours, 20°C, 50,000 rpm) using cesium chloride gradients with propidium iodide was carried out to purify the plasmid DNA. Following centrifugation, the desired plasmid SCP2* DNA band was removed and the propidium iodide extracted by conventional procedures. The CsCl-DNA solution was stored at -20°C. Prior to use, the DNA was desalted by either PD10 (Bio Rad) column exchange with TE or by dialysis against TE. The DNA was precipitated with ethanol by conventional procedures and redissolved in TE.

Example 2

Construction of plasmid pLR1

A. *Hind*III digestion of plasmid pIJ2

About 20 $\mu$l (20 $\mu$g) of plasmid pIJ2 DNA, disclosed in Thompson *et al.,* 1980, Nature 286:525, 5 $\mu$l. BSA (Bovine Serum albumin, 1 mg/ml), 19 $\mu$l water, 1 $\mu$l of *Hind*III (containing 3 New England Bio Labs units) restriction enzyme†††, and 5 $\mu$l reaction mix††††were incubated at 37°C for 2 hours. The reaction was terminated by the addition of about 50 $\mu$l of 4M ammonium acetate and 200 $\mu$l of 95% ethanol. The resultant DNA precipitate was washed twice in 70% ethanol, dried *in vacuo,* suspended in 20 $\mu$l of TE buffer, and frozen at -20°C for storage.

B. *Hind*III Digestion of plasmid pBR322

About 8 $\mu$l (4 $\mu$g) of plasmid pBR322 DNA, 5 $\mu$l reaction mix, 5 $\mu$l BSA (1 mg/ml), 31 $\mu$l water, and 1 $\mu$l of *Hind*III restriction enzyme were incubated at 37°C for 2 hours. After the reaction was terminated by

†Trypticase soy broth is obtained from Difco Laboratories, Detroit, Michigan.

††YEMESG comprises .3% yeast extract, .5% peptone, .3% malt extract, 1% dextrose, 34% sucrose, .1% $MgCl_2$ and .1% glycine.

†††Restriction and other enzymes can be obtained from the following sources:
New England Bio Labs., Inc.
32 Tozer Road
Beverly, Massachusetts 01915
Boerhringer-Mannheim Biochemicals
7941 Castleway Drive
Indianapolis, Indiana 46250
Bethesda Research Laboratories (BRL)

incubating at 60°C for 10 minutes, about 50 μl of ammonium acetate and 200 μl of 95% ethanol were added. The resultant DNA precipitate was washed twice in 70% ethanol, dried *in vacuo,* and suspended in 45 μl of water.

C. Ligation of *Hind*III digested plasmids pIJ2 and pBR322

About 20 μl of *Hind*III treated plasmid pIJ2 (from Example 2A), 20 μl of *Hind*III treated plasmid pBR322 (from Example 2B), 5 μl BSA (1 mg/ml), 1 μl of T4 DNA ligase†, and 5 μl ligation mix††were incubated at 16°C for 4 hours. The reaction was terminated by the addition of about 50 μl 4M ammonium acetate and 200 μl of 95% ethanol. The resultant DNA precipitate was washed twice in 70% ethanol, dried *in vacuo,* and suspended in TE buffer. The suspended DNA constituted the desired plasmid pLR1.

Example 3

Construction of *E. coli* K12 HB101/pLR1

About 10 ml of frozen competent *E. coli* K12 HB101 cells (Bolivar *et al.,* 1977, Gene 2:75—93) were pelleted by centrifugation and then suspended in about 10 ml of .01 M sodium chloride. Next, the cells were pelleted again, resuspended in about 10 ml of .03 M calcium chloride, incubated on ice for 20 minutes, pelleted a third time, and finally, resuspended in 1.25 ml of .03M calcium chloride. The resultant cell suspension was competent for subsequent transformation.

Plasmid pLR1 in TE buffer (prepared in Example 2C) was ethanol precipitated, suspended in 150 μl of 30 mM calcium chloride solution, and gently mixed in a test tube with about 200 μl of competent *E. coli* K12 HB101 cells. The resultant mixture was incubated on ice for about 45 minutes and then at 42°C for about 1 minute. Next, about 3 ml of L-broth (Bertani, 1951, J. Bacteriology 62:293) containing 50 μg/ml of ampicillin was added. The mixture was incubated with shaking at 37°C for 1 hour and then plated on L-agar (Miller, 1972, Experiments in Molecular Genetics, Cold Spring Harbor Labs, Cold Spring Harbor, New York), containing ampicillin. Surviving colonies were selected and tested for the expected phenotype (Amp[R], Tet[S]) and constituted the desired *E. coli* K12 HB101/pLR1 transformants.

Box 6010
Rockville, Maryland 20850
Research Products
Miles Laboratories, Inc.
Elkhart, Indiana 46515

††††Reaction mix for HindIII restriction enzyme was prepared with the following composition:
600 mM NaCl
100 mM Tris-HCl, pH 7.9
70 mM MgCl₂
10 mM Dithiothreitol

†T4 DNA ligase can be obtained from the following source:
New England Bio Labs., Inc.
32 Tozer Rd.
Beverly, Massachusetts 01915

††Ligation mix was prepared with the following composition:
500 mM Tris-HCl, pH 7.8
200 mM Dithiothreitol
100 mM MgCl₂
10 mM ATP

Example 4

Construction of plasmid pLR4

A. Partial *Bam*HI digestion of plasmid pLR1

About 10 $\mu$l (10 $\mu$g) of plasmid pLR1, 5 $\mu$l BSA (1 mg/ml), 29 $\mu$l water, 1 $\mu$l of *Bam*HI (diluted 1:4 with water) restriction enzyme, and 5 $\mu$l reaction mix†††were incubated at 37°C for 15 minutes. The reaction was terminated by the addition of about 50 $\mu$l of 4M ammonium acetate and 200 $\mu$l of 95% ethanol. The resultant DNA precipitate was washed twice in 70% ethanol, dried *in vacuo* and suspended in 20 $\mu$l water.

B. *Bam*HI Digestion of plasmid pBR322

The desired digestion was carried out in substantial accordance with the teaching of Example 2B except that *Bam*HI restriction enzyme and reaction mix were used in place of *Hind*III restriction enzyme and reaction mix. The digested plasmid pBR322 was suspended in 29 $\mu$l of water.

C. Ligation of partial *Bam*HI digested plasmid pLR1 and *Bam*HI digested plasmid pBR322

The desired ligation was carried out in substantial accordance with the teaching of Example 2C. The resultant ligated DNA was suspended in TE buffer and constituted the desired plasmid pLR4.

Example 5

Construction of *E. coli* K12 HB101/pLR4

The desired construction was carried out in substantial accordance with the teaching of Example 3 except that plasmid pLR4, rather than plasmid pLR1, was used for transformation. Surviving colonies were selected and tested for the expected phenotype ($Amp^R$, $Tet^S$) and constituted the desired *E. coli* K12 HB101/pLR4 transformants.

Example 6

Construction of plasmids pJL120 and pJL121

A. *Eco*RI Digestion of plasmid SCP2*

About 150 $\mu$l (5.7 $\mu$g) of plasmid SCP2* DNA, 1 ml water, 2 $\mu$l of *Eco*RI (containing 20 BRL units) restriction enzyme, and 17 $\mu$l. *Eco*RI reaction mix†were incubated at 37°C for 2.5 hours. The reaction was terminated by incubation at 65°C for 15 minutes. The reaction was conventionally analyzed by agarose gel electrophoresis (AGE) to verify that restriction was complete. The restricted DNA was stored at 4°C for subsequent use.

B. *Eco*RI Digestion of plasmid pBR325

The desired digestion was carried out in substantial accordance with the teaching of Example 6A except that plasmid pBR325, rather than plasmid SCP2*, was used. The resultant DNA was stored at 4°C for subsequent use.

†††Reaction mix for BamHI restriction enzyme was prepared with the following composition:
1.5 M NaCl
60 mM Tris-HCl, $p^H$7.9
60 mM $MgCl_2$

†Reaction mix for EcoRI restriction enzyme was prepared with the following composition:
500 mM NaCl
1000 mM Tris-HCl, pH 7.5
100 mM $MgCl_2$

C. Ligation of *Eco*RII digested plasmids SCP2* and pBR325

About 40 $\mu$l of *Eco*RI digested plasmid SCP2* (from Example 6A), 10 $\mu$l of EcoRI digested plasmid pBR325 (from Example 6B), 10 $\mu$l of MgCl$_2$ (.1 M), 10 $\mu$l of (NH$_4$)$_2$SO$_4$ (.1M), 10 $\mu$l ATP (2 mM) .1 $\mu$l of T4 DNA ligase, and 20 $\mu$l ligation mixttwere incubated at 4°C for 18 hours. The reaction was analyzed by AGE to verify appropriate ligation. The suspended DNA constituted the desired ~35.8 kb plasmids pJL120 and pJL121.

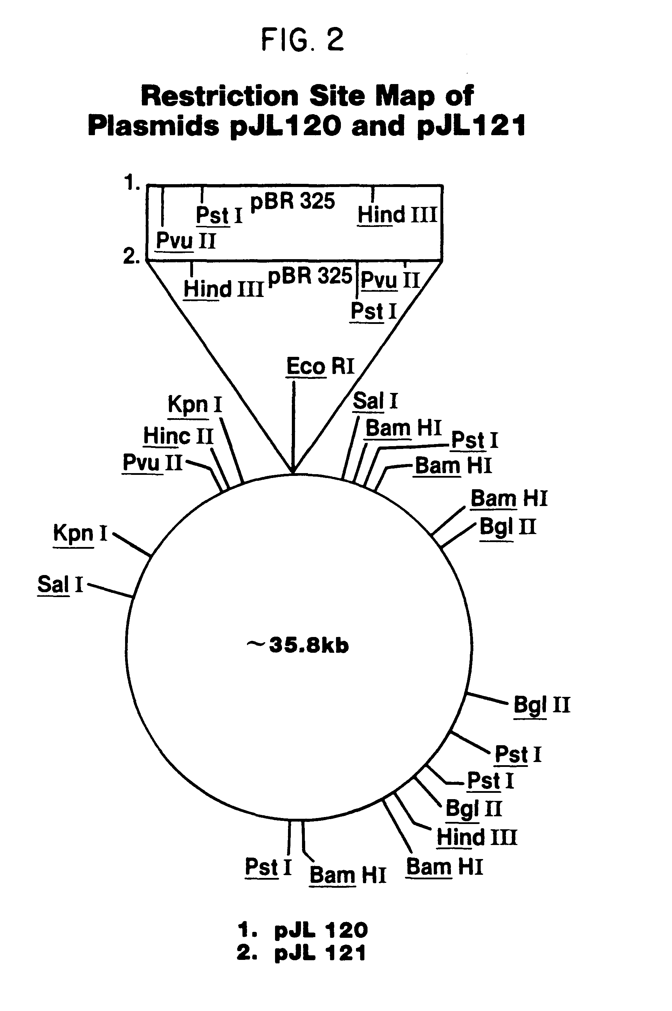

Recombinant plasmids of two orientations result because the plasmid pBR325 *Eco*RI fragment can be oriented in either direction. A restriction site map of each of plasmids pJL120 and pJL121 was determined (after isolation as disclosed in Example 7) and is presented in Figure 2 of the accompanying drawings.

Example 7

Construction of *E. coli* K12 C600R$_k$—M$_k$-/pJL120 and *E. coli* K12 C600R$_k$—M$_k$-/pJL121

A. Preparation of frozen competent *E. coli* K12 C600R$_k$—M$_k$-

Fresh overnight cultures of *E. coli* K12 C600R$_k$—M$_k$- (disclosed in Chang and Cohen, 1974, Proc. Nat. Acad. Sci. 71 :1030—1034) were subcultured 1:10 in fresh L-broth (disclosed in Miller, 1972, Experiments in Molecular Genetics, Cold Spring Harbor Labs, cold Spring Harbor, New York) and grown at 37°C for 1 hour. A total of 660 Klett Units of cells were harvested, washed with 2.5 ml of 100 mM NaCl, suspended in 150 mM CaCl$_2$ with 10% glycerol, and incubated at room temperature for 20 minutes. The cells were harvested by centrifugation, resuspended in .5 ml of CaCl$_2$-glycerol, chilled on ice for 3—5 minutes and frozen. The suspensions of cells were stored in liquid nitrogen until use. Preservation and storage did not adversely affect the viability or frequency of transformation by covalently closed circular DNA.

B. Transformation

The competent cells were thawed in an ice bath and mixed in a ratio of .1 ml of cells to .05 ml of DNA (10 $\mu$l of the sample disclosed in Example 6C and 40 $\mu$l of .1XSSC (.015M NaCl, .0015M Sodium Citrate at pH 7). The transformation mixture was chilled on ice for 20 minutes, heat shocked at 42°C for 1 minute and chilled on ice for 10 minutes. The samples were then diluted with .85 ml of L-broth, incubated at 37°C for 1.5 hours, spread on L-agar containing ampicillin (50 $\mu$g/ml) and tetracycline (12.5 $\mu$g/ml) and incubated for 18 hours at 37°C. The resultant colonies were selected and tested for the expected phenotype (Amp[R], Tet[R], CM[S]) and constituted the desired *E. coli* K12 C600R$_k$—M$_k$-/pJL120 and *E. coli* K12 C600R$_k$—M$_k$-/pJL121 transformants. The ampicillin and tetracycline resistant colonies were isolated according to known procedures, cultured, and then conventionally identified by restriction enzyme and AGE analysis of the constitutive plasmids. The identified transformants were then used for subsequent production and isolation of plasmids pJL120 and pJL121 according to known procedures.

Example 8

Construction of plasmids pJL180 and pJL181

A. *Sal*I Digestion of plasmid SCP2* and isolation of ~6.0 kb *Sal*I fragment

The desired digestion was carried out in substantial accordance with the teaching of Example 6 except

ttLigation mix was prepared with the following composition:
50 mM Tris-HCl, pH 7.5
10 mM $\beta$-mercaptoethanol
1 mM EDTA
50 mg/ml BSA

*Sal*I restriction enzyme and reaction mixt, rather than *Eco*RI restriction enzyme and reaction mix, were used. The reaction was assayed by AGE to verify completion and terminated by heating at 65°C for 15 minutes. The resultant *Sal*I restriction fragments were separated by AGE and then the separated fragments were located in the gel by staining with ethidium bromide and visualizing fluorescent bands with an ultraviolet light. The gel fragment containing the ~6.0 kb fragment of interest was excised from the gel and electroeluted into TBE buffer (1.6% Sigma 7—9 buffertt, .093% $Na_2$EDTA, .55% boric acid). The gel-fragment in TBE buffer was placed in a dialysis bag and subjected to electrophoresis at 100 v for 1 hour. The aqueous solution was collected from the dialysis bag and passed over a DEAE cellulose columnttt(.5 ml Whatman DE52) that had been equilibrated with equilibration buffer (.1 M KCl, 10 mM Tris-HCl, pH 7.8). The column was washed with 2.5 ml of equilibration buffer and the DNA (about 5 $\mu$g) was eluted with 1.5 ml of elution buffer (1M NaCl, 10 mM Tris-HCl, pH 7.8). The eluent was adjusted to about .35M with respect to $Na^+$ ion concentration, and then the DNA was precipitated by adding 2 volumes (about 9 ml) of 100% ethanol followed by cooling to -20°C for 16 hours. The DNA precipitate was pelleted by centrifugation, washed with 75% ethanol, dried, and dissolved in TE buffer. Hereinafter, this conventional isolation technique is referred to as AGE/DE52/electroelution.

B. *Sal*I Digestion of plasmid pBR325

The desired digestion was carried out in substantial accordance with the teaching of Example 8A except that plasmid pBR325 was employed and fragments were not separated by preparative AGE/DE52/electroelution. The resultant DNA was dissolved in TE buffer and stored at 4°C for future use.

C. Ligation of *Sal*I digested plasmid pBR325 and ~6.0 kb *Sal*I fragment of plasmid SCP2*

About 1.5 $\mu$g of the 6.0 kb *Sal*I fragment of SCP2*, prepared in Example 8A, was mixed with .5 $\mu$g of *Sal*I digested pBR325, prepared in Example 8B. The DNA mixture was precipitated by standard ethanol precipitation and redissolved in 3 $\mu$l of distilled water, 4 $\mu$l of .66M ATP, 2 $\mu$l of ligase-kinase mixture (.25M Tris • HCl, pH 7.8, 50 mM $MgCl_2$, 25 mM dithiothreitol and 25% glycerol) and 1 $\mu$l of T4-DNA ligase (1 unit). After incubation for 1 hour at 15°C, the reaction mixture was diluted with 12 $\mu$l of water, 20 $\mu$l of .66M ATP, 8 $\mu$l of ligase-kinase mixture and then incubated at 15°C for 18 hours. The resultant ligated DNA was diluted 1:5 into .1XSSC and constituted the desired ~12.0 kb plasmids pJL180 and pJL181.

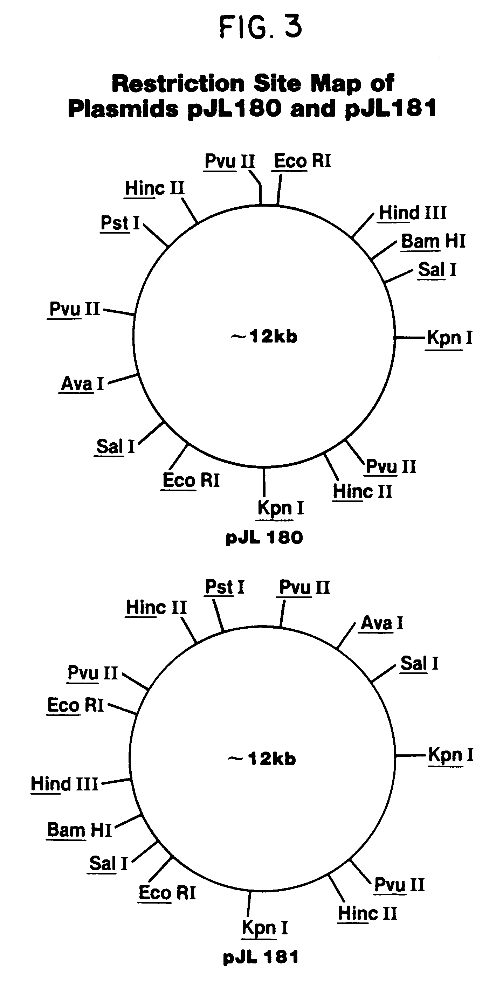

Recombinant plasmids of two orientations result because the plasmid pBR325 *Sal*I fragment can be oriented in either direction. A restriction site map of each of plasmids pJL180 and pJL181 is presented in Figure 3 of the accompanying drawings.

Example 9

Construction of *E. coli* K12 C600$R_k$—$M_k$-/pJL180 and *E. coli* K12 C600$R_k$—$M_k$-/pJL181

The desired constructions were made in substantial accordance with the teaching of Example 7 except that the mixture of plasmid pJL180 and pJL181 DNA (from Example 8C), rather than plasmid pJL120 and pJL121, were used. The resultant transformant colonies were selected and tested for the expected phenotype (Amp$^R$, Tet$^S$, CM$^R$), and constituted the desired *E. coli* K12 C600$R_k$—$M_k$-/pJL180 and *E. coli* K12 C600$R_k$—$M_k$-/pJL181 transformants. The ampicillin and chloramphenicol resistant colonies were isolated according to known procedures, cultured, and then conventionally identified by restriction enzyme and AGE analysis of the constitutive plasmids. The identified transformants can then be used for subsequent production and isolation of plasmids pJL180 and pJL181 according to known procedures.

†Reaction mix for Sall restriction enzyme was prepared with the following composition:
1500 mM NaCl
80 mM Tris-HCl, pH 7.5
60 mM $MgCl_2$
2 mM EDTA

ttSigma 7—9 buffer can be obtained from Sigma Chemical Company, P.O. Box 14508, St. Louis, Missouri 63178

tttDEAE cellulose (DE52) can be obtained from Whatman Inc., 9 Bridewell Place, Clifton, New Jersey 07014.

Example 10

Construction of plasmid pJL125

A. *Sal*I Digestion of plasmid pJL121 and isolation of ~10.2 kb *Sal*I fragment

The desired digestion was carried out in substantial accordance with the teaching of Example 8 except that the reaction was stopped before digestion was complete and except that plasmid pJL121, rather than plasmid SCP2*, was used. The resultant *Sal*I restriction fragments were not separated by preparative AGE but precipitated by standard ethanol precipitation. The restriction fragments were dissolved in TE buffer and immediately ligated.

B. Ligation of ~10.2 kb *Sal*I fragment of plasmid pJL121

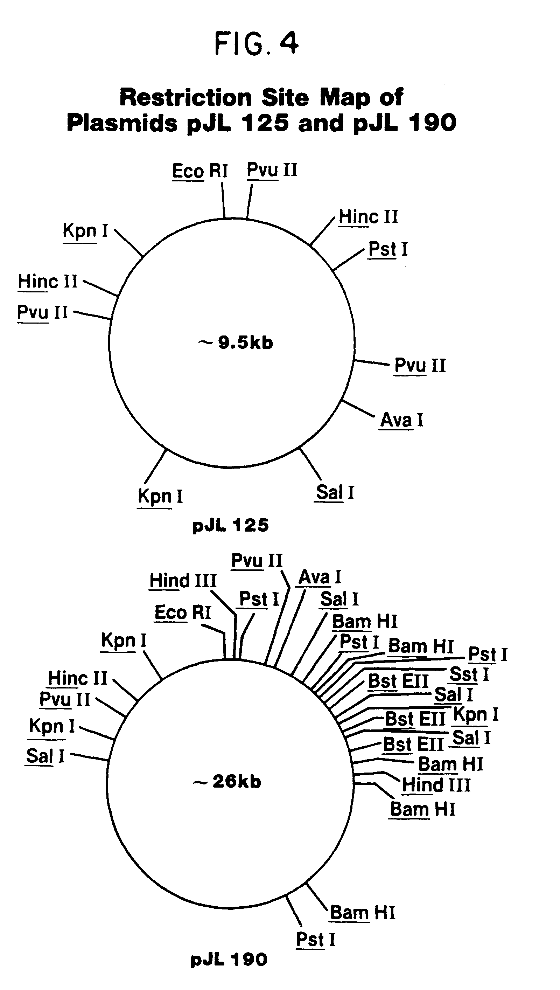

The desired ligation was carried out in substantial accordance with the teaching of Example 8C except that the *Sal*I fragments of plasmid pJL121, rather than the *Sal*I fragment of plasmid SCP2* and pBR325, were used. The resultant ligated DNA constituted the desired plasmid pJL125 plus 12 other plasmids that were subsequently isolated and shown to contain additional *Sal*I restriction fragments of pJL121. Plasmid pJL125, which was conventionally isolated and contains an origin of replication from plasmid pBR325 and also the ~5.4 kb origin of replication-containing *Eco*RI-*Sal*I fragment of plasmid SCP2*, was dissolved in TE buffer and stored at 4°C for future use. A restriction site map of plasmid pJL125 is presented in Figure 4 of the accompanying drawing. The restriction site map was determined with plasmid from transformed *E. coli* K12 C600$R_k$—$M_k$-.

Example 11

Construction of *E. coli* K12 C600$R_k$—$M_k$-/pJL125

The desired construction was made in substantial accordance with the teaching of Example 7 except that plasmid pJL125, rather than plasmids pJL120 and pJL121, was used. The resultant colonies were selected and tested for the expected phenotype (Amp$^R$, Tet$^S$, CM$^S$) and constituted the desired *E. coli* K12 C600$R_k$—$M_k$-/pJL125 transformants. The identity of the transformants was further confirmed by AGE and restriction analysis by the procedure of Eckardt, 1978, Plasmid 1:584 and by Klein *et al.*, 1980, Plasmid 3:88. The transformants were then conventionally cultured for subsequent production and isolation of plasmid pJL125 according to known procedures.

Example 12

Construction of plasmid pJL190

A. *Eco*RI-*Hind*III Digestion of plasmid pJL121 and isolation of ~19.0 kb *Eco*RI-*Hind*III fragment

About 200 μl (80 μg) of plasmid pJL121 DNA, 30 μl BSA (1 mg/ml), 40 μl of *Hind*III (containing 200 BRL units) restriction enzyme, and 30 μl *Hind*III reaction mix† were incubated at 37°C for about 3 hours and then at 65°C for 10 minutes. The 300 μl reaction mixture was cooled to 4°C, supplemented with 110 μl of 10× *Hind*III→*Eco*RI diluent reaction mix†† and 30 μl *Eco*RI restriction enzyme (containing 300 BRL units), and then incubated at 37°C for 3 hours, then at 65°C for 10 minutes followed by cooling to 4°C. The resultant ~19.0 kb *Eco*RI-*Hind*III restriction fragment was conventionally isolated by AGE/DE52/electroelution. The desired DNA was dissolved in TE buffer and stored at 4°C for future use.

†HindIII reaction mix was prepared with the following composition:
60 mM Tris-HCl, pH 7.5
500 mM NaCl
60 mM MgCl₂

B. *Eco*RI-*Hind*III Digestion of plasmid pLR4 and isolation of ~7.7 kb *Eco*RI-*Hind*III fragment

The desired digestion and isolation was carried out in substantial accordance with the teaching of Example 12A except that plasmid pLR4, rather than plasmid pJL121, was used. The desired ~7.7 kb fragment was dissolved in TE buffer and stored at 4°C for future use.

C. Ligation of ~19.0 kb *Eco*RI-*Hind*III fragment of plasmid pJL121 and ~7.7 kb *Eco*RI-*Hind*III fragment of plasmid pLR4

The desired ligation was carried out in substantial accordance with the teaching of Example 8C except that the ~19.0 kb *Eco*RI-*Hind*III fragment of plasmid pJL121 and the ~7.7 *Eco*RI-*Hind*III fragment of plasmid pLR4, rather than the 6.0 kb *Sal*I fragment of plasmid SCP2* and *Sal*I digested pBR325, were used. The resultant ligated DNA constituted the desired plasmid pJL190 which was then stored at 4°C for future use. A restriction site and functional map of plasmid pJL190 is presented in Figure 4 of the accompanying drawings. The restriction site map was determined from plasmid transformed into *E. coli* K12 C600$R_k$—$M_k$-.

Example 13

Construction of *E. coli* K12 C600$R_k$—$M_k$-/pJL190

The desired construction was made in substantial accordance with the teaching of Example 7 except that plasmid pJL190, rather than plasmids pJL120 and pJL121, was used. The resultant colonies were selected and tested for the expected phenotype (Amp$^R$, Tet$^S$) and size by conventional means (as in Example 11) and constituted the desired *E. coli* K12 C600$R_k$—$M_k$-/pJL190 transformants. The transformants were then conventionally cultured for subsequent production and isolation of plasmid pJL190 according to known procedures.

Example 14

Isolation of plasmid pJL192

Plasmid pJL192, which confers high resistance to antibiotic neomycin (10 $\mu$g/ml), can be conventionally isolated from *E. coli* K12 C600$R_k$—$M_k$-/pJL192, a strain deposited and made part of the permanent stock culture collection of the Northern Regional Research Laboratory, Peoria, Illinois under the accession number 15040. The restriction site map of plasmid pJL192 appears not be distinguishable from the plasmid pJL190 map presented in Figure 4.

Example 15

Construction of plasmid pJL195

A. *Eco*RI-*Sal*I Digestion of plasmid pJL125 and isolation of ~5.4 kb *Eco*RI-*Sal*I fragment

The desired digestion and isolation was carried out in substantial accordance with the teaching of Example 12A except that plasmid pJL125 and *Sal*I restriction enzyme and reaction mix, rather than plasmid pJL121 and *Hind*III restriction enzyme and reaction mix, were used. In addition, *Sal*I→*Eco*RI diluent†was used. The resultant ~5.4 kb *Eco*RI-*Sal*I fragment was dissolved in TE buffer and stored at 4°C for future use.

††HindIII→EcoRI diluent was prepared with the following composition:
382 mM Tris-HCl, pH 7.5
50 mM NaCl
22 mM MgCl₂

†SalI→EcoRI diluent was prepared with the following composition:
940 mM Tris-HCl, pH 7.5
55 mM MgCl₂

B. *Eco*RI-Partial *Sal*I digestion of plasmid pLR4 and isolation of ~7.5 kb *Eco*RI-partial *Sal*I fragment

The *Sal*I digestion was carried out in substantial accordance with the teaching of Example 12A except plasmid pLR4, rather than pJL121, was used. Since only a partial *Sal*I digestion was desired, the resultant mixture was incubated first at 37°C for 15 minutes and then at 65°C for 10 minutes. Following cooling to 4°C, the resultant partial *Sal*I~7.7 kb linear fragment was conventionally isolated by AGE/DE52/electroelution. The desired DNA was dissolved in TE buffer and digested with *Eco*RI restriction enzyme in substantial accordance with the teaching of Example 6A except that the above fragment, rather than the *Sal*I fragments of SCP2*, was used. The desired ~7.5 kb *Eco*RI-*Sal*I fragment (the largest possible *Eco*RI-*Sal*I fragment) was isolated by AGE/DE52/electroelution, dissolved in TE buffer, and then stored at 4°C for future use.

C. Ligation of ~5.4 kb *Eco*RI-*Sal*I fragment of plasmid pJL125 and ~7.5 kb *Eco*RI-Partial *Sal*I fragment of plasmid pLR4

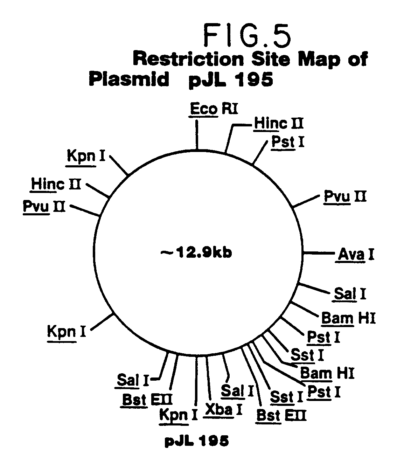

The desired ligation was carried out in substantial accordance with the teaching of Example 8C except that the ~5.4 kb *Eco*RI-*Sal*I fragment of plasmid pJL125 and the ~7.5 kb *Eco*RI-partial *Sal*I fragment of plasmid pLR4, rather than the ~6.0 kb *Sal*I fragment of plasmid SCP2* and *Sal*I digested pBR325, were used. The resultant ligated DNA constituted the desired plasmid pJL195 and was stored at 4°C for future use. A restriction site map of plasmid pJL195 is presented in Figure 5 of the accompanying drawings. The restriction site map was determined with plasmid isolation from *E. coli* K12 C600R$_k$—M$_k$-.

Example 16

Construction of *E. coli* K12 C600R$_k$—M$_k$-/pJL195

The desired construction was made in substantial accordance with the teaching of Example 7 except that plasmid pJL195, rather than plasmids pJL120 and pJL121, was used. The resultant colonies were tested for the expected phenotype (Amp$^R$, Tet$^S$) and size (as in Example 11) and constituted the desired *E. coli* K12 C600R$_k$—M$_k$-/pJL195 transformants. The transformants were then conventionally cultured for subsequent production and isolation of plasmid pJL195 according to known procedures.

Example 17

Construction of *Streptomyces griseofuscus*/pJL120

A. Growth of cultures for preparation of protoplasts

A vegetative inoculum was conventionally prepared by growing the strain under submerged conditions for 20 hours at 30°C in TSB supplemented with .4% glycine. The culture was homogenized and inoculated at a 1/20 dilution into the same medium and then grown for 18 hours at 30°C.

B. Transformation

Using about 20 μg of plasmid pJL120 DNA and 1 × 10⁹ protoplasts of *Streptomyces griseofuscus,* a strain deposited and made part of the permanent stock culture collection of the American Type Culture Collection, Rockville, Maryland, from which it is available to the public under the accession number ATCC 23916, the desired transformation was carried out in substantial accordance with the teaching of International Publication (of International Patent Application No. PCT/GB79/00095) No. W079/01169, Example 2.

C. Selection

To assay for transformation even at low frequencies, two procedures were employed.

(1) Pock-assay;

Spores were harvested from the regeneration plates containing confluent lawns of regenerated protoplasts as follows. About 10 ml of sterile distilled water was added to the plate and the surface of the culture

17

gently scraped with a loop to remove the spores. The resulting spore suspension was centrifuged at 20,000 rpm for 10 minutes. The supernatant was discarded and the remaining spore pellet resuspended in .3 ml of 20% v/v glycerol. Serial dilutions of the preparation were made down to $10^{-5}$ by successive transfer of .1 ml of the spore suspension to .9 ml of 20% v/v glycerol. The spores can then be stored at -20°C with little loss of viability. About .1 ml aliquots of some of the dilution series (e.g. $10^{-1}$, $10^{-2}$, $10^{-4}$) of each of the harvested plates were then transferred to R2 medium (Hopwood and Wright, 1978, Molecular and General Genetics 162:30) plates which had sufficient spores of the *Streptomyces griseofuscus* strain originally used in the transformation procedure to produce a confluent lawn. This procedure can also be carried out with the substitution of YMX agar (.5% yeast extract .5% malt extract, .1% dextrose and 2% agar). Transformants can typically be detected after 3 days' growth at 30°C by the appearance of "pocks", a property expressed by spores containing the plasmid in expressible form within the lawn. The transformants were recovered by conventionally picking spores from the centre of the "pock" to an agar plate of YMX medium (Hopwood 1967, Bacteriological Review, 31:373).

(2) Back transformation to *E. coli* K12 $C600R_k$—$M_k$-:

The spores are collected as in (1) above but are used to inoculate 50 ml of TSB supplemented with .4% glycine. The culture is grown for 20 hours at 30°C and the cells are harvested followed by isolation of DNA. Isolation is as disclosed in Example 1B except that centrifugation with CsCl and propidium iodide is omitted. Subsequently, 50 $\mu$l of this DNA is used to transform *E. coli* K12 $C600R_k$—$M_k$- as disclosed in Example 7B. Plasmids in the transformants are verified and identified by conventional means as taught in Example 11.

Example 18

Construction of *S. griseofuscus*/pJL121, *S. griseofuscus*/pJL125, *S. griseofuscus*/pJL180, and *S. griseofuscus*/pJL181.

The desired constructions were each individually and respectively made, selected, and recovered in substantial accordance with the teaching of Example 17 except that plasmids pJL121, pJL125, pJL180, and pJL181, rather than plasmid pJL120, were appropriately used for the individual construction.

Example 19

Construction of *Streptomyces griseofuscus*/pJL190

A. Transformation

The desired transformation was carried out in substantial accordance with the teaching of Example 17B except that plasmid pJL190, rather than plasmid pJL120, was used.

B. Selection

The desired transformants were selected for neomycin resistance by overlaying the regenerating protoplasts with R2 medium top agar containing sufficient neomycin to bring the final plate concentration to 1 $\mu$g/ml. The resultant *Streptomyces griseofuscus*/pJL190 transformants were then tested for the expected pock morphology in substantial accordance with the procedure of Example 17C(2).

Example 20

Construction of *Streptomyces griseofuscus/*pJL195

The desired construction was made, selected, and recovered in substantial accordance with the teaching of Example 19 except that plasmid pJL195, rather than plasmid pJL190, was used.

18

Example 21

Construction of *Streptomyces griseofuscus*/pJL192

The desired construction was made, selected, and recovered in substantial accordance with the teaching of Example 20 except that plasmid pJL192 and, in the selection procedure, top agar containing sufficient neomycin to bring the final plate concentration to 10 $\mu$g/ml, rather than plasmid pJL195 and top agar containing sufficient neomycin to bring the final plate concentration to 1 $\mu$g/ml, were used.

Example 22

Construction of *Streptomyces fradiae*/pJL120, *S. fradiae/pJL121, S. fradiae/pJL 125,* S. fradiae/pJL180, *S. fradiae*/pJL181, *S. fradiae*/pJL190, *S. fradiae*/pJL195, and *S. fradiae*/pJL192

The desired constructions are individually and respectively made, selected, and recovered in substantial accordance with the respective teachings of Examples 18, 19, 20, and 21 except that *Streptomyces fradiae,* rather than *S. griseofuscus,* is used. In addition, the TSB medium for protoplasting and growing *S. fradiae* was modified and contained only .2% glycine.

Example 23

Construction of *Streptomyces lividans*/pJL120, *S. lividans*/pJL121, *S. lividans*/pJL125, S. lividans/pJL180, *S. lividans/pJL181,* S. lividans/pJL190, *S. lividans*/pJL195, and *S. lividans/pJL192*

The desired constructions are individually and respectively made, selected, and recovered in substantial accordance with the respective teachings of Examples 18, 19, 20, and 21 except that *Streptomyces lividans,* rather than *S. griseofuscus,* is used. In addition, the media for protoplasting and growing *S. lividans* is as described in International Publication (of International Patent Application No. PCT/GB79/00095) No. WO79/01169, Example 2.

Example 24

Construction of plasmid pLR2

A. *Hind*III Digestion of plasmid pIJ6

About 20 $\mu$l (20 $\mu$g) of plasmid pIJ6 DNA, disclosed in Thompson, *et al.,* 1980, Nature 286:525,5 $\mu$l BSA (Bovine Serum albumin, 1 mg/ml), 19 $\mu$l water, 1 $\mu$l of *Hind*III (containing 3 New England Bio Labs units) restriction enzyme*, and 5 $\mu$l reaction mix* were incubated at 37°C for 2 hours. The reaction was terminated by the addition of about 50 $\mu$l of 4M ammonium acetate and 200 $\mu$l of 95% ethanol. The resultant DNA precipitate was washed twice in 70% ethanol, dried *in vacuo,* suspended in 20 $\mu$l of TE buffer, and frozen at -20°C for storage.

B. *Hind*III Digestion of plasmid pBR322

About 8 $\mu$l (4 $\mu$g) of plasmid pBR322 DNA, 5 $\mu$l reaction mix, 5 $\mu$l BSA (1 mg/ml) 31 $\mu$l water, and 1 $\mu$l of *Hind*III restriction enzyme were incubated at 37°C for 2 hours. After the reaction was terminated by incubating at 60°C for 10 minutes, about 50 $\mu$l of ammonium acetate and 200 $\mu$l of 95% ethanol were added. The resultant DNA precipitate was washed twice in 70% ethanol, dried *in vacuo,* and suspended in 45 $\mu$l of water.

C. Ligation of *Hind*III digested plasmids pIJ6 and pBR322

About 20 $\mu$l of *Hind*III treated plasmid pIJ6 (from Example 2A), 20 $\mu$l of *Hind*III treated plasmid pBR322 (from Example 2B), 5 $\mu$l BSA (1 mg/ml), 1 $\mu$l of T4 DNA ligase†, and 5 $\mu$l ligation mixt were incubated at 16°C for 4 hours. The reaction was terminated by the addition of about 50 $\mu$l 4M ammonium acetate and

†See Example 2

200 $\mu$l of 95% ethanol. The resultant DNA precipitate was washed twice in 70% ethanol, dried *in vacuo,* and suspended in TE buffer. The suspended DNA constituted the desired plasmid pLR2.

Example 25

Construction of *E. coli* K12 HB101/pLR2

About 10 ml of *E. coli* K12 HB101 cells (Bolivar *et al.,* 1977, Gene 2:75—93) were pelleted by centrifugation and then suspended in about 10 ml of 0.01M sodium chloride. Next, the cells were pelleted again, resuspended in about 10 ml of 0.03M calcium chloride, incubated on ice for 20 minutes, pelleted a third time, and finally, resuspended in 1.25 ml of 0.03M calcium chloride. The resultant cell suspension was competent for subsequent transformation.

Plasmid pLR2 in TE buffer was ethanol precipitated, suspended in 150 $\mu$l of 30 mM calcium chloride solution, and gently mixed in a test tube with about 200 $\mu$l of competent *E. coli* K12 HB101 cells. The resultant mixture was incubated on ice for about 45 minutes and then at 42°C for about 1 minute. Next, about 3 ml of L-broth (Bertani, 1951, J. Bacteriology 62:293) containing 50 $\mu$g/ml of ampicillin were added. The mixture was incubated with shaking at 37°C for 1 hour and then plated on L-agar (Miller, 1972, Experiments in Molecular Genetics, Cold Spring Harbor Labs, Cold Spring Harbor, New York) containing ampicillin. Surviving colonies were selected and tested for the expected phenotype (Amp[R], Tet[S]), and constituted the desired *E. coli* K12 HB101/pLR2 transformants.

Representative plasmids and transformants that can be constructed in accordance with the foregoing teaching include the following listed below in Tables 1 and 2.

TABLE 1

| Representative plasmids | | | | | |
|---|---|---|---|---|---|
| Example No. | Plasmid name | ~Size in kb | *E. coli* marker | *Streptomyces* marker | Construction |
| 26 | pJL123 | 27.8 | Amp[R], Tet[R] | M | *Bgl*II Deletion of pJL121 |
| 27 | pJL1200 | 35.8 | Amp[R], Tet[R] | M | SCP2 *Eco*RI site into pBR325 *Eco*RI site |
| 28 | pJL1201 | 35.8 | Amp[R], Tet[R] | M | Insertional isomer of pJL1200 |
| 29 | pJL1203 | 27.8 | Amp[R], Tet[R] | M | *Bgl*II Deletion of pJL1201 |
| 30 | pJL1205 | 10.2 | Amp[R] | M | *Sal*I Deletion of pJL1201 |
| 31 | pJL1716 | 22.5 | CM[R,] Tet[R] | P | ~16.6 *Pst*I of SCP2 into *Pst*I site of pBR325 |
| 32 | pJL3176 | 22.5 | CM[R], Tet[R] | P* | ~16.6 *Pst*I of SCP2* into *Pst*I site of pBR325 |
| 33 | pJL1800 | 12.6 | Amp[R], CM[R] | P | ~6.0 kb *Sal*I of SCP2 into *SSsal*I site of pBR325 |
| 34 | pJL1801 | 12.6 | Amp[R], CM[R] | P | Insertional isomer of pJL1800 |
| 35 | pJL1900 | 25.9 | Amp[R] | M, Neo[R] | ~1910 kb *Eco*RI-*Hind*III of pJL1201 and ~7.7 kb *Eco*RI-*Hind*III of pLR4 |
| 36 | pJL1905 | 12.1 | Amp[R] | M, Neo[R] | ~5.4 kb *Eco*RI-*Sal*I of pJL1205 and ~ 7.5 kb *Eco*RI-Partial *Sal*I of pPR4 |

20

TABLE 2

Representative transformants

1. *Streptomyces* R/pR1† wherein R is *griseofuscus, ambofaciens, fradiae,* or *lividans* and R1 is independently pJL123, pJL1200, pJL1201, pJL1203, pJL1205, pJL1800, pJL1801, pJL1900, pJL1905, and pJL196.
2. *E. coli* K12 $R^2$/pR$^1$ wherein $R^2$ is C600$R_k$—$M_k$-, 294, C600 or RV308 and R$^1$ is independently as defined above.

†The expression R/pR$^1$ indicates that *Streptomyces* species R is transformed with a plasmid selected from the group R$^1$.

**Claims**
**Claims for the following Contracting States : BE, CH, DE, FR, GB, IT, LI, LU, NL, SE**

1. A recombinant DNA cloning vector which has as the only restriction fragment derived from plasmid SCP2 or SCP2* a ~5.4 kb EcoRI-SalI or ~6.0 kb SalI restriction fragment comprising an origin of replication functional in Streptomyces, and which further comprises
   a) a restriction fragment comprising the functional origin of a multi-copy E.coli plasmid,
   b) one or more DNA segments that confer resistance to at least one antibiotic when transformed into a cell of E. coli, said cell being sensitive to the antibiotic for which resistance is conferred, and
   c) one or more DNA segments that confer resistance to at least one antibiotic when transformed into a cell of Streptomyces, said cell being sensitive to the antibiotic for which resistance is conferred.

2. The vector of Claim 1 which is a plasmid.

3. The vector of Claim 1 or 2 wherein the multicopy E.coli plasmid is plasmid pBR322, pBR324, pBR325, pBR327, or pBR328.

4. The vector of any of Claims 1 to 3 wherein the one or more DNA segments that confer resistance in E. coli are DNA segments that confer resistance to ampicillin, chloramphenicol or tetracycline.

5. The vector of any of Claims 1 to 4 wherein the one or more DNA segments that confer resistance in Streptomyces are DNA segments that confer resistance to neomycin or thiostrepton.

6. The vector of Claim 5 which is

| Plasmid | ~ Size in kb | E. coli Marker | Streptomyces Marker | Construction |
|---------|------|----------------|---------------------|--------------|
| pJL195 | 12.9 | Amp$^R$, Tet$^S$ | M, Neo$^R$ | Inserting the EcoRI digest of SCP2* into the EcoRI site of pBR325 to give pJL121, self-ligating the ~10.2 kb SalI fragment of pJL121 to give pJL125, and ligating the ~5.4 kb EcoRI-SalI fragment of pJL125 and the ~7.5 kb EcoRI-partial SalI digest of pLR4. |

7. The vector of Claim 5 which is

| Plasmid | ~ Size in kb | E. coli Marker | Streptomyces Marker | Construction |
|---|---|---|---|---|
| pJL1905 | 12.1 | Amp$^R$ | M, Neo$^R$ | Inserting the EcoRI digest of SCP2 into the EcoRI site of pBR325 to give pJL1201, self-ligating the ~10.2 kb SalI fragment of pJL1201 to give pJL1205, and ligating the ~5.4 kb EcoRI-SalI fragment of pJL1205 and the ~7.5 kb EcoRI-partial SalI digest of pLR4. |

8. The vector of Claim 5 which is

| Plasmid | ~ Size in kb | E. coli Marker | Streptomyces Marker | Construction |
|---------|--------------|----------------|---------------------|--------------|
| pJL196 | 12.1 | Amp$^R$, | M, Neo$^R$ | Ligation of the ~5.4 kb EcoRI-SalI of pJL125 into the ~7.5 kb EcoRI-partial SalI fragment of pJL192 (NRRL B-15040). |

9. The vector of any of Claims 1 to 8 wherein the DNA segment that confers resistance to an antibiotic is the ~7.7 kb EcoRI-HindIII restriction fragment of plasmid pJL192 (NRRL B-15040), the ~7.7 kb EcoR-HindIII restriction fragment of plasmid pLR4, the ~7.5 kb EcoRI-partial SalI restriction fragment of plasmid pLR4, or the ~1.35 kb BamHI restriction fragment of plasmid pLR2.

10. A transformed host cell comprising the recombinant DNA cloning vector of any of Claims 1 to 9.

11. The host cell of Claim 10 which is Streptomyces preferably of species lividans, griseofuscus, fradiae, or ambofaciens.

12. The host cell of Claim 10 which is E. coli K12.

13. A restriction fragment which is the plasmid SCP2 or SCP2* ~5.4 kb EcoRI-SalI fragment or the ~6.0 kb SalI fragment.

14. A restriction fragment comprising the fragment of Claim 13 and the ~7.7 kb EcoRI-HindIII fragment of plasmid pJL192 (NRRL B-15040).

15. A process for preparing a recombinant DNA cloning vector which has as the only restriction fragment derived from plasmid SCP2 or SCP2* a ~5.4 kb EcoRI-SalI or ~6.0 kb SalI restriction fragment comprising an origin of replication functional in Streptomyces, which comprises ligating the said fragment, and
    a) a restriction fragment comprising the functional origin of a multi-copy E. coli plasmid,
    b) one or more DNA segments that confer resistance to at least one antibiotic when transformed into a cell of E. coli, said cell being sensitive to the antibiotic for which resistance is conferred, and
    c) one or more DNA segments that confer resistance to at least one antibiotic when transformed into a cell of Streptomyces, said cell being sensitive to the antibiotic for which resistance is conferred.

16. The process of Claim 15 wherein the origin of replication of the multicopy E. coli plasmid is the pBR322, pBR324, pBR325, pBR327 or pBR328 origin of replication.

17. The process of Claim 15 or 16 wherein the one or more DNA segments that confer resistance in E. coli are DNA segments that confer resistance to ampicillin, chloramphenicol or retracycline and wherein the one or more DNA segments that confer resistance in Streptomyes are DNA segments that confer resistance to neomycin or thiostrepton.

18. A method for detecting transformants which comprises:
    1) mixing Streptomyces cells, under transforming conditions, with a recombinant DNA cloning vector comprising
        a) an origin of replication and P gene-containing restriction fragment of plasmid SCP2 or SCP2*, and
        b) a non-lethal DNA sequence cloned into the EcoRI restriction site of the P gene, and
    2) growing the Streptomyces cells on a lawn of an indicator Streptomyces strain and selecting colonies that show the M pock phenotype.

**19.** The method of Claim 18 wherein the restriction fragment comprising the origin of replication and the P gene is of plasmid SCP2*.

**20.** The method of Claim 18 and 19 wherein the Streptomyces cells and indicator strain are preferably of species lividans, griseofuscus, fradiae or ambofaciens.

**21.** The method of Claim 19 or 20 wherein the recombinant DNA cloning vector is plasmid pJL120 (Figure 2), pJL121 (Figure 2), pJL190 (Figure 4), pJL192 (NRRL B-15040) or pJL195 (Figure 5).

**Claims for the following Contracting State : AT**

**1.** A process for preparing a recombinant DNA cloning vector which has as the only restriction fragment derived from plasmid SCP2 or SCP2* a ~5.4 kb EcoRI-SalI or ~6.0 kb SalI restriction fragment comprising an origin of replication functional in Streptomyces, which comprises ligating the said fragment, and
   a) a restriction fragment comprising the functional origin of a multi-copy E. coli plasmid,
   b) one or more DNA segments that confer resistance to at least one antibiotic when transformed into a cell of E. coli, said cell being sensitive to the antibiotic for which resistance is conferred, and
   c) one or more DNA segments that confer resistance to at least one antibiotic when transformed into a cell of Streptomyces, said cell being sensitive to the antibiotic for which resistance is conferred.

**2.** The process of Claim 1 wherein the multicopy E. coli plasmid is plasmid pBR322, pBR324, pBR325, pBR327, or pBR328.

**3.** The process of Claim 1 or 2 wherein the one or more DNA segments that confer resistance in E. coli are DNA segments that confer resistance to ampicillin, chloramphenicol or tetracycline.

**4.** The process of any of Claims 1 to 3 wherein the one or more DNA segments that confer resistance in Streptomyces are DNA segments that confer resistance to neomycin or thiostrepton.

**5.** The process of Claim 4 for preparing

24

| Plasmid | ~ Size in kb | E. coli Marker | Streptomyces Marker | Construction |
|---|---|---|---|---|
| pJL1905 | 12.1 | Amp$^R$ | M, Neo$^R$ | Inserting the EcoRI digest of SCP2 into the EcoRI site of pBR325 to give pJL1201, self-ligating the ~10.2 kb SalI fragment of pJL1201 to give pJL1205, and ligating the ~5.4 kb EcoRI-SalI fragment of pJL1205 and the ~7.5 kb EcoRI-partial SalI digest of pLR4. |

6. The vector of any of Claims 1 to 5 wherein the DNA segment that confers resistance to an antibiotic is the ~7.7 kb EcoRI-HinIII restriction fragment of plasmid pJL192 (NRRL B-15040), the ~7.7 kb EcoRI-HindIII restriction fragment of plasmid pLR4, the ~7.5 kb EcoRI-partial SalI restriction fragment of plasmid pLR4, or the ~1.35 kb BamHI restriction fragment of plasmid pLR2.

7. The process of any of Claims 1 to 4 for preparing plasmid pJL195 which comprises inserting the EcoRI digest of SCP2* into the EcoRI site of pBR325 to give pJL121, self-ligating the ~10.2 kb SalI fragment of pJL121 to give pJL125, and ligating the ~5.4 kb EcoRI-SalI fragment of pJL125 and the ~7.5 kb EcoRI-partial SalI digest of pLR4.

8. A transformed host cell comprising a recombinant DNA cloning vector prepared in accordance with any of Claims 1 to 7.

9. The host cell of Claim 8 which is Streptomyces, preferably of species lividans, griseofuscus, fradiae, or ambofaciens.

10. The host cell of Claim 8 which is E. coli K12.

**Patentansprüche**
**Patentansprüche für folgende Vertragsstaaten : BE, CH, DE, FR, GB, IT, LI, LU, NL, SE**

1. Rekombinanter DNA-Klonierungsvektor, der als einziges Restriktionsfragment, das sich von Plasmid SCP2 oder SCP2* ableitet, ein ˜5,4 kb-EcoRI-SalI- oder ˜6,0 kb-SalI-Restriktionsfragment, das einen in Streptomyces funktionellen Replikationsursprung enthält, aufweist, und weiter umfaßt:
   a) ein Restriktionsfragment mit der funktionellen Ursprungsstelle eines Multikopien-E. coli-Plasmids,
   b) ein oder mehrere DNA-Segmente, die Resistenz gegenüber mindestens einem Antibiotikum verleihen, wenn sie in eine Zelle von E. coli transformiert werden, wobei die Zelle gegenüber dem Antibiotikum, gegen das Resistenz verliehen wird, empfindlich ist, und
   c) ein oder mehrere DNA-Segmente, die Resistenz gegen mindestens ein Antibiotikum verleihen, wenn sie in eine Zelle von Streptomyces transformiert werden, wobei die Zelle gegenüber dem Antibiotikum, gegen das Resistenz verliehen wird, empfindlich ist.

2. Vektor nach Anspruch 1, bei dem es sich um ein Plasmid handelt.

3. Vektor nach Anspruch 1 oder 2, wobei es sich beim Multikopien-E. coli-Plasmid um das Plasmid pBR322, pBR324, pBR325, pBR327 oder pBR328 handelt.

4. Vektor nach einem der Ansprüche 1 bis 3, wobei es sich bei dem oder den DNA-Segmenten, die in E. coli Resistenz verleihen, um DNA-Segmente handelt, die Resistenz gegen Ampicillin, Chloramphenicol oder Tetracyclin verleihen.

5. Vektor nach einem der Ansprüche 1 bis 4, wobei es sich bei dem oder den DNA-Segmenten, die Resistenz in Streptomyces verleihen, um DNA-Segmente handelt, die Resistenz gegen Neomycin oder Thiostrepton verleihen.

6. Vektor nach Anspruch 5, bei dem es sich um folgendes handelt:

| Plasmid | ~Größe in kb | E. coli-Marker | Streptomyces Marker | Konstruktion |
|---|---|---|---|---|
| pJL195 | 12,9 | Amp$^R$, Tet$^S$ | M, Neo$^R$ | Insertion des EcoRI-Verdaus von SCP2* in die EcoRI-Stelle von pBR325, was pJL121 ergibt, Selbstligation des ~10,2 kb-SalI-Fragmentes von pJL121, was pJL125 ergibt, und Ligation des ~5,4 kb-EcoRI-SalI-Fragments von pJL125 und des ~7,5 kb-EcoRI-partiellen SalI-Verdauungsprodukts vom pLR4. |

7. Vektor nach Anspruch 5, bei dem es sich um folgendes handelt:

| Plasmid | ~Größe in kb | E. coli-Marker | Streptomyces Marker | Konstruktion |
|---|---|---|---|---|
| pJL1905 | 12,1 | Amp$^R$ | M, Neo$^R$ | Insertion des EcoRI-Verdaus von SCP2 in die EcoRI-Stelle von pBR325, was pJL1201 ergibt, Selbstligation des ~10,2 kb-SalI-Fragmentes von pJL1201, was pJL1205 ergibt, und Ligation des ~5,4 kb-EcoRI-SalI-Fragments von pJL1205 und des ~7,5 kb-EcoRI-partiellen SalI-Verdaus von pLR4. |

8. Vektor nach Anspruch 5, bei dem es sich um folgendes handelt:

| Plasmid | ~Größe in kb | E. coli-Marker | Streptomyces Marker | Konstruktion |
|---|---|---|---|---|
| pJL196 | 12,1 | Amp$^R$ | M, Neo$^R$ | Ligation des ~5,4 kb-EcoRI-SalI von pJL125 in das ~7,5 kb-EcoRI-partielle SalI-Fragment von pJL192 (NRRL B-15040). |

9. Vektor nach einem der Ansprüche 1 bis 8, wobei es sich bei dem DNA-Segment, das Resistenz gegen ein Antibiotikum verleiht, um das ~7,7 kb-EcoRI-HindIII-Restriktionsfragment des Plasmids pJL192 (NRRL B-15040), das ~7,7 kb-EcoRI-HindIII-Restriktionsfragment des Plasmids pLR4, das ~7,5 kb-EcoRI-partielle SalI-Restriktionsfragment des Plasmids pLR4 oder das ~1,35 kb-BamHI-Restriktionsfragment des Plasmids pLR2 handelt.

10. Transformierte Wirtszelle mit einem Gehalt an dem rekombinanten DNA-Klonierungsvektor nach einem der Ansprüche 1 bis 9.

11. Wirtszelle nach Anspruch 10, bei der es sich um Streptomyces, vorzugsweise der Spezies lividans, griseofuscus, fradiae oder ambofaciens, handelt.

12. Wirtszelle nach Anspruch 10, wobei es sich um E. coli K12 handelt.

13. Restriktionsfragment, das das ~5,4 kb-EcoRI-SalI-Fragment oder das ~6,0 kb-SalI-Fragment aus dem Plasmid SCP2 oder SCP2* ist.

14. Restriktionsfragment, enthaltend das Fragment von Anspruch 13 und das ~7,7 kb-EcoRI-HindIII-Fragment von Plasmid pJL192 (NRRL B-15040).

15. Verfahren zur Herstellung eines rekombinanten DNA-Klonierungsvektors, der als einziges Restriktionsfragment, das sich von Plasmid SCP2 oder SCP2* ableitet, ein ~5,4 kb-EcoRI-SalI-oder ~6,0 kb-SalI-Restriktionsfragment enthält, das einen in Streptomyces funktionellen Replikationsursprung umfaßt, umfassend die Ligation dieses Fragments mit
a) einem Restriktionsfragment mit der funktionellen Ursprungsstelle eines Multikopien-E. coli-Plasmids,
b) einem oder mehreren DNA-Segmenten, die Resistenz gegenüber mindestens einem Antibiotikum verleihen, wenn sie in eine Zelle von E. coli transformiert werden, wobei die Zelle gegenüber dem Antibiotikum, gegen das Resistenz verliehen wird, empfindlich ist, und
c) einem oder mehreren DNA-Segmenten, die Resistenz gegen mindestens ein Antibiotikum verleihen, wenn sie in eine Zelle von Streptomyces transformiert werden, wobei die Zelle gegenüber dem Antibiotikum, gegen das Resistenz verliehen wird, empfindlich ist.

16. Verfahren nach Anspruch 15, wobei es sich bei der Replikationsursprungsstelle des Multikopien-E. coli-Plasmids um die pBR322-, pBR324-, pBR325-, pBR327- oder pBR328-Replikationsursprungsstelle handelt.

17. Verfahren nach Anspruch 15 oder 16, wobei es sich bei dem oder den DNA-Segmenten, die Resistenz in E. coli verleihen, um DNA-Segmente handelt, die Resistenz gegen Ampicillin, Chloramphenicol oder Tetracyclin verleihen und wobei es sich bei dem oder den DNA-Segmenten, die Resistenz in Streptomyces verleihen, um DNA-Segmente handelt, die Resistenz gegen Neomycin oder Thiostrepton verleihen.

18. Verfahren zum Nachweis von Transformanten, umfassend
1) Das Vermischen von Streptomyces-Zellen unter Transformationsbedingungen mit einem rekombinanten DNA-Klonierungsvektor, enthaltend

a) ein eine Replikationsursprungsstelle und das P-Gen enthaltendes Restriktionsfragment des Plasmids SCP2 oder SCP2* und

b) eine nicht-letale DNA-Sequenz, die in die EcoRI-Restriktionsstelle des P-Gens geklont ist und

2) das Züchten der Streptomyces-Zellen auf einem Rasen eines Indikator-Streptomyces-Stamms und das Auswählen der Kolonien, die den M-Pustel-Phänotyp zeigen.

19. Verfahren nach Anspruch 18, wobei das die Replikationsursprungsstelle und das P-Gen enthaltende Restriktionsfragment aus Plasmid SCP2* stammt.

20. Verfahren nach Anspruch 18 oder 19, wobei die Streptomyces-Zellen und der Indikator-Stamm vorzugsweise zur Spezies lividans, griseofuscus, fradiae oder ambofaciens gehören.

21. Verfahren nach Anspruch 19 oder 20, wobei es sich bei dem rekombinanten DNA-Klonierungsvektor um das Plasmid pJL120 (Figur 2), pJL121 (Figur 2), pJL190 (Figur 4), pJL192 (NRRL B-15040) oder pJL195 (Figur 5) handelt.

**Patentansprüche für folgenden Vertragsstaat : AT**

1. Verfahren zur Herstellung eines rekombinanten DNA-Klonierungsvektors, der als einziges Restriktionsfragment, das von Plasmid SCP2 oder SCP2* abgeleitet ist, ein ˜5,4 kb-EcoRI-SalI-oder ˜6,0 kb-SalI-Restriktionsfragment enthält, das einen in Streptomyces funktionellen Replikationsursprung umfaßt, umfassend die Ligation dieses Fragments mit

a) einem Restriktionsfragment mit der funktionellen Ursprungsstelle eines Multikopien-E. coli-Plasmids,

b) einem oder mehreren DNA-Segmenten, die Resistenz gegenüber mindestens einem Antibiotikum verleihen, wenn sie in eine Zelle von E. coli transformiert werden, wobei die Zelle gegenüber dem Antibiotikum, gegen das Resistenz verliehen wird, empfindlich ist, und

c) einem oder mehreren DNA-Segmenten, die Resistenz gegen mindestens ein Antibiotikum verleihen, wenn sie in eine Zelle von Streptomyces transformiert werden, wobei die Zelle gegenüber dem Antibiotikum, gegen das Resistenz verliehen wird, empfindlich ist.

2. Verfahren nach Anspruch 1, wobei es sich bei dem Multikopien-E. coli-Plasmid um das Plasmid pBR322, pBR324, pBR325, pBR327 oder pBR328 handelt.

3. Verfahren nach Anspruch 1 oder 2, wobei es sich bei dem oder den DNA-Segmenten, die Resistenz in E. coli verleihen, um DNA-Segmente handelt, die Resistenz gegen Ampicillin, Chloramphenicol oder Tetracyclin verleihen.

4. Verfahren nach einem der Ansprüche 1 bis 3, wobei es sich bei dem oder den DNA-Segmenten, die Resistenz in Streptomyces verleihen, um DNA-Segmente handelt, die Resistenz gegen Neomycin oder Thiostrepton verleihen.

5. Verfahren nach Anspruch 4 zur Herstellung von

| Plasmid | ~Größe in kb | E. coli-Marker | Streptomyces Marker | Konstruktion |
|---------|--------------|----------------|---------------------|--------------|
| pJL1905 | 12,1 | $Amp^R$ | M, $Neo^R$ | Insertion des EcoRI-Verdaus von SCP2 in die EcoRI-Stelle von pBR325, was pJL1201 ergibt, Selbstligation des ~10,2 kb-SalI-Fragmentes von pJL1201, was pJL1205 ergibt, und Ligation des ~5,4 kb-EcoRI-SalI-Fragments von pJL1205 und des ~7,5 kb-EcoRI-partiellen SalI-Verdaus von pLR4. |

6. Vektor nach einem der Ansprüche 1 bis 5, wobei es sich bei dem DNA-Segment, das Resistenz gegen ein Antibiotikum verleiht, um das ˜7,7 kb-EcoRI-HindIII-Restriktionsfragment des Plasmids pJL192 (NRRL B-15040), das ˜7,7 kb-EcoRI-HindIII-Restriktionsfragment des Plasmids pLR4, das ˜7,5 kb-EcoRI-partielle SalI-Restriktionsfragment des Plasmids pLR4 oder das ˜135 kb-BamHI-Restriktionsfragment des Plasmids pLR2 handelt.

7. Verfahren nach einem der Ansprüche 1 bis 4 zur Herstellung des Plasmids pJL195, das die Insertion des EcoRI-Verdaus von SCP2* in die EcoRI-Stelle von pBR325, was pJL121 ergibt, die Selbstligation des ˜10,2 kb-SalI-Fragments von pJL121, was pJL125 ergibt, und die Ligation des ˜5,4 kb-EcoRI-SalI-Fragments von pJL125 mit dem ˜7,5 kb-EcoRI-partiellen SalI-Verdau von pLR4 umfaßt.

8. Transformierte Wirtszelle, enthaltend einen rekombinanten DNA-Klonierungsvektor, der nach einem der Ansprüche 1 bis 7 hergestellt ist.

9. Wirtszelle nach Anspruch 8, wobei es sich um Streptomyces, vorzugsweise der Gattung lividans, griseofuscus, fradiae oder ambofaciens, handelt.

10. Wirtszelle nach Anspruch 9, wobei es sich um E. coli K12 handelt.

**Revendications**
**Revendications pour les Etats contractants suivants : BE, CH, DE, FR, GB, IT, LI, LU, NL, SE**

1. Vecteur de clonage d'ADN recombinant qui possède, comme fragment de restriction unique dérivé du plasmide SCP2 ou SCP2*, un fragment de restriction EcoRI-SalI d'environ 5,4 kb ou un fragment de restriction SalI d'environ 6,0 kb, comprenant une origine de réplication fonctionnelle dans Streptomyces, et qui comprend, en outre
   a) un fragment de restriction comprenant l'origine fonctionnelle d'un plasmide E. coli multicopie,
   b) un ou plusieurs segments d'ADN conférant de la résistance à au moins un antibiotique après transformation en une cellule de E. coli, ladite cellule étant sensible à l'antibiotique pour lequel on a conféré de la résistance, et
   c) un ou plusieurs segments d'ADN qui confèrent de la résistance à au moins un antibiotique après transformation en une cellule de Streptomyces, ladite cellule étant sensible à l'antibiotique pour lequel on a conféré de la résistance.

2. Vecteur selon la revendication 1, ce vecteur étant un plasmide.

3. Vecteur selon la revendication 1 ou 2, dans lequel le plasmide E. coli multicopie est le plasmide pBR322, pBR324, pBR325, pBR327 ou pBR328.

4. Vecteur selon l'une quelconque des revendications 1 à 3, dans lequel le ou les segments d'ADN conférant de la résistance dans E. coli sont des segments d'ADN conférant de la résistance à l'ampicilline, au chloramphénicol ou à la tétracycline.

5. Vecteur selon l'une quelconque des revendications 1 à 4, dans lequel le ou les segments d'ADN conférant de la résistance dans Streptomyces sont des segments d'ADN conférant de la résistance à la néomycine ou à la thiostreptone.

6. Vecteur selon la revendication 5, ce vecteur étant

| Plasmide | pJL195 |
|---|---|
| Taille en kb | 12,9 |
| Marqueur E. coli | Amp$^R$, Tet$^S$ |
| Marqueur Streptomyces | M, Neo$^R$ |
| Construction | insérer le produit de digestion de SCP2* par EcoRI dans le site EcoRI de pBR325 pour obtenir pJL121, procéder à l'autoligature du fragment SalI d'environ 10,2 kb de pJL121 pour obtenir pJL125, et ligaturer le fragment EcoRI-SalI d'environ 5,4 kb de pJL125 et le produit de digestion partielle SalI par EcoRI d'environ 7,5 kb de pLR4. |

7. Vecteur selon la revendication 5, ce vecteur étant

| Plasmide | pJL1905 |
|---|---|
| Taille en kb | 12,1 |
| Marqueur E. coli | Amp$^R$ |
| Marqueur Streptomyces | M, Neo$^R$ |
| Construction | insérer le produit de digestion de SCP2 par EcoRI dans le site EcoRI de pBR325 pour obtenir pJL1201, procéder à l'autoligature du fragment SalI d'environ 10,2 kb de pJL1201 pour obtenir pJL1205, e t ligaturer le fragment EcoRI-SalI d'environ 5,4 kb de pJL1205 et le produit de digestion partielle SalI par EcoRI d'environ 7,5 kb de pLR4. |

8. Vecteur selon la revendication 5, ce vecteur étant

| Plasmide | pJL196 |
|---|---|
| Taille en kb | 12,1 |
| Marqueur E. coli | Amp$^R$ |
| Marqueur Streptomyces | M, Neo$^R$ |
| Construction | ligature du fragment EcoRI-SalI d'environ 5,4 kb de pJL125 dans le fragment SalI partiel-EcoRI d'environ 7,5 kb de pJL192 (NRRL B-15040). |

9. Vecteur selon l'une quelconque des revendications 1 à 8, dans lequel le segment d'ADN qui confère de la résistance à un antibiotique est le fragment de restriction EcoRI-HindIII d'environ 7,7 kb du plasmide pJL192 (NRRL B-15040), le fragment de restriction EcoRI-HindIII d'environ 7,7 kb du plasmide pLR4, le fragment de restriction SalI partiel-EcoRI d'environ 7,5 kb du plasmide pLR4, ou le fragment de restriction BamHI d'environ 1,35 kb du plasmide pLR2.

10. Cellule hôte transformée comprenant le vecteur de clonage d'ADN recombinant selon l'une quelconque des revendications 1 à 9.

11. Cellule hôte selon la revendication 10, ladite cellule étant une cellule Streptomyces, de préférence, de l'espèce lividans, griseofuscus, fradiae ou ambofaciens.

12. Cellule hôte selon la revendication 10, ladite cellule étant E. coli K12.

13. Fragment de restriction, à savoir le fragment EcoRI-SalI d'environ 5,4 kb du plasmide SCP2 ou SCP2*, ou le fragment SalI d'environ 6,0 kb.

14. Fragment de restriction comprenant le fragment selon la revendication 13 et le fragment EcoRI-HindIII d'environ 7,7 kb du plasmide pJL192 (NRRL B-15040).

**15.** Procédé de préparation d'un vecteur de clonage d'ADN recombinant qui possède, comme fragment de restriction unique dérivé du plasmide SCP2 ou SCP2*, un fragment de restriction EcoRI-SalI d'environ 5,4 kb ou un fragment de restriction SalI d'environ 6,0 kb, comprenant une origine de réplication fonctionnelle dans Streptomyces, qui comprend le fait de ligaturer ledit fragment et

    a) un fragment de restriction comprenant l'origine fonctionnelle d'un plasmide E. coli multicopie,

    b) un ou plusieurs segments d'ADN conférant de la résistance à au moins un antibiotique après transformation en une cellule de E. coli, ladite cellule étant sensible à l'antibiotique pour lequel on a conféré de la résistance, et

    c) un ou plusieurs segments d'ADN qui confèrent de la résistance à au moins un antibiotique après transformation en une cellule de Streptomyces, ladite cellule étant sensible à l'antibiotique pour lequel on a conféré de la résistance.

**16.** Procédé selon la revendication 15, dans lequel l'origine de réplication du plasmide E. coli multicopie est l'origine de réplication de pBR322, pBR324, pBR325, pBR327 ou pBR328.

**17.** Procédé selon la revendication 15 ou 16, dans lequel le ou les segments d'ADN conférant de la résistance dans E. coli, sont des segments d'ADN conférant de la résistance à l'ampicilline, au chloramphénicol ou à la tétracycline, et dans lequel le ou les segments d'ADN conférant de la résistance dans Streptomyces sont des segments d'ADN conférant de la résistance à la néomycine ou à la thiostreptone.

**18.** Procédé de détection de transformants, qui comprend le fait de

    1) mélanger des cellules Streptomyces, dans des conditions de transformation, avec un vecteur de clonage d'ADN recombinant, ce vecteur comprenant :

        a) une origine de réplication, ainsi qu'un fragment de restriction contenant le gène P du plasmide SCP2 ou SCP2*, et

        b) une séquence d'ADN non léthale clonée dans le site de restriction EcoRI du gène P, et

    2) faire croître les cellules Streptomyces sur une "pelouse" d'une souche Streptomyces à indicateur et sélectionner des colonies qui présentent le phénotype "M pock".

**19.** Procédé selon la revendication 18, dans lequel le fragment de restriction comprenant l'origine de réplication et le gène P est un fragment de restriction du plasmide SCP2*.

**20.** Procédé selon les revendications 18 et 19, dans lequel les cellules Streptomyces et la souche à indicateur sont, de préférence, de l'espèce lividans, griseofuscus, fradiae ou ambofaciens.

**21.** Procédé selon la revendication 19 ou 20, dans lequel le vecteur de clonage d'ADN recombinant est le plasmide pJL120 (figure 2), pJL121 (figure 2), pJL190 (figure 4), pJL192 (NRRL B-15040) ou pJL195 (figure 5).

**Revendications pour l'Etat contractant suivant : AT**

**1.** Procédé de préparation d'un vecteur de clonage d'ADN recombinant qui possède, comme fragment de restriction unique dérivé du plasmide SCP2 ou SCP2*, un fragment de restriction EcoRI-SalI d'environ 5,4 kb ou un fragment de restriction SalI d'environ 6,0 kb, comprenant une origine de réplication fonctionnelle dans Streptomyces, qui comprend le fait de ligaturer ledit fragment et

    a) un fragment de restriction comprenant l'origine fonctionnelle d'un plasmide E. coli multicopie,

    b) un ou plusieurs segments d'ADN conférant de la résistance à au moins un antibiotique après transformation en une cellule de E. coli, ladite cellule étant sensible à l'antibiotique pour lequel on a conféré de la résistance, et

    c) un ou plusieurs segments d'ADN qui confèrent de la résistance à au moins un antibiotique après transformation en une cellule de Streptomyces, ladite cellule étant sensible à l'antibiotique pour lequel on a conféré de la résistance.

**2.** Procédé selon la revendication 1, dans lequel le plasmide multicopie E. coli est le plasmide pBR322, pBR324, pBR325, pBR327 ou pBR328.

3. Procédé selon la revendication 1 ou 2, dans lequel le ou les segments d'ADN conférant de la résistance dans <u>E. coli</u> sont des segments d'ADN conférant de la résistance à l'ampicilline, au chloramphénicol ou à la tétracycline.

4. Procédé selon l'une quelconque des revendications 1 à 3, dans lequel le ou les segments d'ADN conférant de la résistance dans <u>Streptomyces</u> sont des segments d'ADN conférant de la résistance à la néomycine ou à la thiostreptone.

5. Procédé selon la revendication 4, pour préparer

| <u>Plasmide</u> | pJL1905 |
|---|---|
| <u>Taille en kb</u> | 12,1 |
| <u>Marqueur E. coli</u> | Amp$^R$ |
| <u>Marqueur Streptomyces</u> | M, Neo$^R$ |
| <u>Construction</u> | insérer le produit de digestion de SCP2 par <u>Eco</u>RI dans le site <u>Eco</u>RI de pBR325 pour obtenir pJL1201, procéder à l'autoligature du fragment <u>Sal</u>I d'environ 10,2 kb de pJL1201 pour obtenir pJL1205, e t ligaturer le fragment <u>Eco</u>RI-<u>Sal</u>I d'environ 5,4 kb de pJL1205 et le produit de digestion partielle <u>Sal</u>I par <u>Eco</u>RI d'environ 7,5 kb de pLR4. |

6. Vecteur selon l'une quelconque des revendications 1 à 5, dans lequel le segment d'ADN qui confère de la résistance à un antibiotique est le fragment de restriction <u>Eco</u>RI-<u>Hin</u>dIII d'environ 7,7 kb du plasmide pJL192 (NRRL B-15040), le fragment de restriction <u>Eco</u>RI-<u>Hin</u>dIII d'environ 7,7 kb du plasmide pLR4, le fragment de restriction <u>Sal</u>I partiel-<u>Eco</u>RI d'environ 7,5 kb du plasmide pLR4, ou le fragment de restriction <u>Bam</u>HI d'environ 1,35 kb du plasmide pLR2.

7. Procédé selon l'une quelconque des revendications 1 à 4, pour préparer le plasmide pJL195, qui comprend le fait d'insérer le produit de digestion de SCP2* par <u>Eco</u>RI dans le site <u>Eco</u>RI de pBR325 pour obtenir pJL121, procéder à l'autoligature du fragment <u>Sal</u>I d'environ 10,2 kb de pJL121 pour obtenir pJL125, et ligaturer le fragment <u>Eco</u>RI-<u>Sal</u>I d'environ 5,4 kb de pJL125 et le produit de digestion partielle <u>Sal</u>I par <u>Eco</u>RI d'environ 7,5 kb de pLR4.

8. Cellule hôte transformée comprenant un vecteur de clonage d'ADN recombinant préparé conformément à l'une quelconque des revendiations 1 à 7.

9. Cellule hôte selon la revendication 8, ladite cellule étant une cellule <u>Streptomyces</u>, de préférence, de l'espèce <u>lividans</u>, <u>griseofuscus</u>, <u>fradiae</u> ou <u>ambofaciens</u>.

10. Cellule hôte selon la revendication 8, ladite cellule étant <u>E. coli</u> K12.

36

## FIG. I

## Restriction Site Map of Plasmids SCP2 and SCP2*

SCP2 and SCP2*

# FIG. 2

## Restriction Site Map of Plasmids pJL120 and pJL121

1. pJL 120
2. pJL 121

# FIG. 3

## Restriction Site Map of
## Plasmids pJL180 and pJL181

pJL 180

pJL 181

# FIG. 4

## Restriction Site Map of
## Plasmids pJL 125 and pJL 190

pJL 125

pJL 190

# FIG.5

## Restriction Site Map of
## Plasmid pJL 195

pJL 195